# EUROPÄISCHE PATENTSCHRIFT

(11) **EP 3 459 509 B1**
(45) Veröffentlichungstag und Bekanntmachung des Hinweises auf die Patenterteilung: **07.06.2023**
(21) Anmeldenummer: 17020431.7
(22) Anmeldetag: 20.09.2017
(51) Int. Cl.: A61F 9/008

(54) **OPHTHALMOLOGISCHE VORRICHTUNG ZUM BEARBEITEN VON AUGENGEWEBE MITTELS EINES GEPULSTEN BEARBEITUNGSLASTERSTRAHLS**
OPHTHALMOLOGICAL DEVICE FOR TREATING EYE TISSUE USING A PULSED PROCESSING LASER BEAM
DISPOSITIF OPHTALMOLOGIQUE DE TRAITEMENT DE TISSU OCULAIRE À L'AIDE D'UN RAYON LASER D'USINAGE PULSÉ

(43) Veröffentlichungstag der Anmeldung: 27.03.2019
(73) Patentinhaber: Ziemer Ophthalmic Systems AG, 2562 Port (CH)
(72) Erfinder: Rathjen, Christian, 28197 Bremen (DE)
(74) Vertreter: Rentsch Partner AG

(56) Entgegenhaltungen:
- EP-A1- 2 596 775
- EP-A1- 2 990 015
- WO-A1-2016/049442
- WO-A2-2006/074469
- DE-A1-102008 051 644

## Beschreibung

### Technisches Gebiet

Die vorliegende Erfindung betrifft eine ophthalmologische Vorrichtung zum Bearbeiten von Augengewebe mittels eines Bearbeitungslaserstrahls, der durch ein Scannersystem fokussiert ins Augengewebe gelenkt wird.

### Stand der Technik

Zum Bearbeiten von Augengewebe mittels eines Laserstrahls wird ein Bearbeitungsbereich mit Laserpulsen gescannt (abgetastet), indem der gepulste Laserstrahl mittels geeigneter Scannersysteme (Ablenkvorrichtungen) in ein oder zwei Scannrichtungen (Abtastrichtungen) abgelenkt wird. Die Ablenkung der Lichtstrahlen respektive der Laserpulse, beispielsweise Femtosekundenlaserpulse, wird im Allgemeinen mit beweglichen Spiegeln vorgenommen, die um eine oder zwei Scannachsen schwenkbar sind, beispielsweise mit Galvanoscannern, Piezoscannern, Polygonscannern oder Resonanzscannern.

In US 7,621,637 wird eine Vorrichtung zum Bearbeiten von Augengewebe beschrieben, welche eine Basisstation mit einer Laserquelle zur Erzeugung von Laserpulsen und einen in der Basisstation angeordneten Scanner mit beweglichen Ablenkspiegeln zum Ablenken der Laserpulse in einer Scannrichtung aufweist. Die abgelenkten Laserpulse werden über ein optisches Übertragungssystem von der Basisstation zu einem Applikationskopf übertragen, der mittels einer mechanisch bewegten Projektionsoptik einen Arbeitsbereich gemäss einem Scannmuster abfährt. Die im Vergleich zur mechanischen Bewegung viel schnellere Ablenkung in der Scannrichtung wird im Applikationskopf auf die mechanische Bewegung der Projektionsoptik und somit auf dessen Scannmuster überlagert. Ein Fast-Scannersystem in der Basisstation ermöglicht eine feine Bewegung der Laserpulse (Microscann), welche auf das Scannmuster der beweglichen Projektionsoptik überlagert wird, die einen grossen Bearbeitungsbereich abdeckt, beispielsweise das gesamte Auge.

Derartige bekannte Systeme ermöglichen zwar die Bearbeitung von einfachen Scannmustern, beispielsweise das Schneiden eines Gewebelappens (Flap), das in der Regel als grosses Flächenstück mit einfacher Randgeometrie ausgeführt wird. Bei Anwendungen, in denen nicht nur Gewebeschnitte in einer im Wesentlichen horizontal ausgerichteten Bearbeitungsfläche auf einer gemeinsamen Fokalfläche ausgeführt werden, sondern in denen auch Schnitte mit einer vertikalen Schnittkomponente über unterschiedliche Fokushöhen ausgeführt werden sollen, z.B. schräg zur Horizontalen verlaufende oder vertikale Schnitte, um beispielsweise im Augengewebe Lentikel zu schneiden, erweist sich das vertikale Verfahren der Projektionsoptik oder eines Zoom-Systems für eine vertikale Veränderung des Fokus und damit der Schnitthöhe als zu langsam um Schnitte mit einer vertikalen Komponente, also mit veränderlicher Fokustiefe während des Schneidens, mit einer Geschwindigkeit auszuführen, die vergleichbar ist mit Schnittgeschwindigkeiten in der horizontalen Bearbeitungsfläche.

US 2016/0089270 beschreibt ein System und Verfahren zum Schneiden von Lentikeln im Augengewebe. Gemäss US 2016/0089270 werden dazu gradlinige Fastscannlinien langsameren Bearbeitungslinien überlagert, die entlang Meridianen des Lentikels abgefahren werden. Durch die Gradlinigkeit der Fastscannlinien entstehen Schnitte, die in ihrer Form von der gewünschten Oberflächenkrümmung des Lentikels abweichen und daher Fehler verursachen. Überdies ist zum Abfahren der Bearbeitungslinien entlang der Meridiane jeweils über die Distanz einer Lentikelbreite eine vertikale Fokusverschiebung in der Grössenordnung und im Ausmass der Dicke des zu schneidenden Lentikels erforderlich, was einerseits mit entsprechendem Aufwand und Kosten für dazu eingerichtete verschiebbare Optiken und bewegliche Linsen und andererseits mit damit einhergehenden Einbussen in der Bearbeitungsgeschwindigkeit verbunden ist. Darüber hinaus erlauben die Fastscannlinien aufgrund ihrer festen horizontalen Ausrichtung keine bestmögliche Anpassung von Schnitten an Lentikeloberflächen, insbesondere nicht, wenn diese von einer sphärischen Form abweichen. Zudem ist zu beachten, dass beim Schneiden von Lentikeloberflächen in unterschiedlichen Tiefenlagen Vorkehrungen getroffen werden müssen, um einerseits Abschattungen von tieferliegenden Lagen durch höher positionierte Schnitte zu vermeiden, aber andererseits die Präzision und Genauigkeiten der Schnitte nicht zu beeinträchtigen und die Bearbeitungszeit möglichst nicht unnötig zu verlängern.

Bei diesen bekannten Systemen werden sequenziell zuerst eine untere Fläche eines Lentikels und danach eine obere Fläche des Lentikels geschnitten. Bewegt sich zwischen diesen beiden Schritten das Augengewebe oder folgt die Fokusführung nicht genau vorgegebenen Schnittkoordinaten, dann ergibt sich eine Abweichung in der Dicke des Lentikels, was zu refraktiven Fehlern führt.

In 2006/0195076 wird eine ophthalmologische Vorrichtung zum Bearbeiten von Augenlinsengewebe beschrieben, welche ein Multifokalsystem umfasst, das eingerichtet ist, aus dem gepulsten Laserstrahl den Bearbeitungslaserstrahl derart zu erzeugen, dass der Bearbeitungslaserstrahl einen ersten Strahlteil und einen zweiten Strahlteil umfasst, welche durch eine Fokussieroptik auf unterschiedliche Flächen im Augengewebe fokussiert werden, wobei beide Flächen unterschiedlich tief liegen. Hier handelt es sich jedoch nicht um ein System, das geeignet wäre, Lentikel zu schneiden.

### Darstellung der Erfindung

Es ist eine Aufgabe der vorliegenden Erfindung eine ophthalmologische Vorrichtung zum Bearbeiten von Augengewebe mittels eines Bearbeitungslaserstrahls vorzuschlagen, welche zumindest einige Nachteile der bekannten Systeme nicht aufweist. Es ist insbesondere eine Aufgabe der vorliegenden Erfindung eine ophthalmologische Vorrichtung zum Bearbeiten von Augengewebe mittels eines Bearbeitungslaserstrahls vorzuschlagen, welche ein effizientes Schneiden von Lentikeln im Augengewebe ermöglicht.

Gemäss der vorliegenden Erfindung werden diese Ziele durch die Merkmale der unabhängigen Ansprüche erreicht. Weitere vorteilhafte Ausführungsformen gehen ausserdem aus den abhängigen Ansprüchen und der Beschreibung hervor.

Eine ophthalmologische Vorrichtung zum Bearbeiten von Augengewebe umfasst eine Laserquelle, die eingerichtet ist, einen gepulsten Laserstrahl zu erzeugen, eine Fokussieroptik, die eingerichtet ist, einen Bearbeitungslaserstrahl ins Augengewebe zu fokussieren, und ein Scannersystem, das eingerichtet ist, den Bearbeitungslaserstrahl gemäss vorgegebenen Scannmustern ins Augengewebe zu lenken.

Die oben genannten Ziele werden durch die vorliegende Erfindung insbesondere dadurch erreicht, dass die ophthalmologische Vorrichtung zudem ein Strahlteilsystem umfasst, das dem Scannersystem vorgeschaltet ist und das eingerichtet ist, aus dem gepulsten Laserstrahl den Bearbeitungslaserstrahl derart zu erzeugen, dass der Bearbeitungslaserstrahl einen ersten Strahlteil und einen zweiten Strahlteil umfasst, wobei der erste Strahlteil durch die Fokussieroptik auf eine erste Aussenfläche eines im Augengewebe zu schneidenden Lentikels fokussiert wird und der zweite Strahlteil auf eine andere zweite Aussenfläche des im Augengewebe zu schneidenden Lentikels fokussiert wird, und dass bei einem Lenken des Bearbeitungslaserstrahl ins Augengewebe durch das Scannersystem die erste Aussenfläche durch den ersten Strahlteil bearbeitet wird und die zweite Aussenfläche durch den zweiten Strahlteil bearbeitet wird. Die Erzeugung des Bearbeitungslaserstrahls mit zwei Strahlteilen, die ohne Verstellung der Fokussieroptik sowohl auf die tiefer gelegene untere Aussenfläche des zu schneidenden Lentikels als auch auf die vergleichsweise höher gelegene obere Aussenfläche des Lentikels fokussiert wird, ermöglicht ein effizienteres, schnelleres Schneiden des Lentikels, da mit jeder Einstellung der Fokussieroptik und des Scannersystems gleichzeitig die untere und die obere Aussenfläche des Lentikels bearbeitet werden kann. Überdies werden durch gleichzeitiges Schneiden der unteren und oberen Aussenflächen des Lentikels unerwünschte Abweichungen von der Solldicke des Lentikels reduziert, die sich aufgrund von Bewegungen des Gewebes während des Schneidvorgangs ergeben können.

In einer Ausführungsvariante umfasst das Strahlteilsystem einen dem Scannersystem vorgeschalteten Divergenzmodulator, der eingerichtet ist, eine Divergenz des ersten Strahlteils oder des zweiten Strahlteils zu modulieren, so dass der erste Strahlteil und der zweite Strahlteil eine unterschiedliche Divergenz aufweisen, derart dass der erste Strahlteil durch die Fokussieroptik auf die erste Aussenfläche fokussiert wird und der zweite Strahlteil auf die zweite Aussenfläche fokussiert wird. Der dem Scannersystem vorgeschalteten Divergenzmodulator und die dadurch bewirkte Divergenzmodulation zumindest eines der Strahlteile ermöglicht die Erzeugung eines Bearbeitungslaserstrahls mit einem Dualfokus zur Bearbeitung zweier Aussenflächen eines Lentikels in unterschiedlichen Tiefenlagen, ohne dafür die Fokussieroptik oder das Scannersystem anders einstellen zu müssen. Mit anderen Worten, die Einstellung der Fokussieroptik und des Scannersystems zur Bearbeitung eines Arbeitspunkts im Augengewebe, bewirkt die Bearbeitung an zwei verschiedenen Bearbeitungspunkten im Augengewebe, einer auf der unteren Aussenfläche des Lentikels und einen auf der oberen Aussenfläche des Lentikels.

In einer Ausführungsvariante umfasst das Strahlteilsystem einen ersten Divergenzmodulator, der eingerichtet ist, den ersten Strahlteil mit einer ersten Divergenz zu erzeugen, und einen zweiten Divergenzmodulator, der eingerichtet ist, den zweiten Strahlteil mit einer von der ersten Divergenz verschiedenen zweiten Divergenz zu erzeugen.

In einer Ausführungsvariante ist die Fokussieroptik eingerichtet, den Bearbeitungslaserstrahl im Augengewebe auf eine eingestellte Schnittposition zu fokussieren. Der erste Divergenzmodulator ist eingerichtet, den ersten Strahlteil derart mit der ersten Divergenz zu erzeugen, dass der erste Strahlteil durch die Fokussieroptik im Augengewebe relativ zur eingestellten Schnittposition auf die erste Aussenfläche fokussiert wird, und der zweite Divergenzmodulator ist eingerichtet, den zweiten Strahlteil derart mit der zweiten Divergenz zu erzeugen, dass der zweite Strahlteil durch die Fokussieroptik im Augengewebe relativ zur eingestellten Schnittposition auf die zweite Aussenfläche fokussiert wird.

In einer Ausführungsvariante umfasst das Strahlteilsystem einen Strahlteiler, der eingerichtet ist, den gepulsten Laserstrahl in ein erstes Strahlenbündel und in ein zweites Strahlenbündel zu teilen.

In einer Ausführungsvariante ist der Strahlteiler eingerichtet, einen ersten Puls des gepulsten Laserstrahls dem ersten Strahlenbündel zuzuführen, und einen dem ersten Puls unmittelbar folgenden zweiten Puls des gepulsten Laserstrahls dem zweiten Strahlenbündel zuzuführen.

In einer Ausführungsvariante umfasst der Strahlteiler einen optischen Modulator, der synchronisiert mit der Laserquelle gekoppelt ist und eingerichtet ist, den ersten Puls des gepulsten Laserstrahls dem ersten Strahlenbündel zuzuführen, und den zweiten Puls des gepulsten Laserstrahls dem zweiten Strahlenbündel zuzuführen.

In einer Ausführungsvariante umfasst der Strahlteiler einen optischen Strahlteiler, der eingerichtet ist, den gepulsten Laserstrahl in das erste Strahlenbündel und in das zweite Strahlenbündel aufzuteilen. Der Strahlteiler umfasst einen ersten optischen Modulator, der synchronisiert mit der Laserquelle gekoppelt im ersten Strahlenbündel angeordnet ist und eingerichtet ist, den zweiten Puls des gepulsten Laserstrahls im ersten Strahlenbündel auszublenden. Der Strahlteiler umfasst einen zweiten optischen Modulator, der synchronisiert mit der Laserquelle gekoppelt im zweiten Strahlenbündel angeordnet ist und eingerichtet ist, den ersten Puls des gepulsten Laserstrahls im zweiten Strahlenbündel auszublenden.

In einer Ausführungsvariante umfasst das Strahlteilsystem einen Strahlvereiniger, der eingerichtet ist, das erste Strahlenbündel und das zweite Strahlenbündel zum Bearbeitungslaserstrahl zu vereinen.

In einer Ausführungsvariante ist der Strahlvereiniger eingerichtet, das erste Strahlenbündel und das zweite Strahlenbündel mit einem Winkel zwischen einer Strahlachse des ersten Strahlenbündels und einer Strahlachse des zweiten Strahlenbündels zum Bearbeitungslaserstrahl zu vereinen. Der Bearbeitungslaserstrahl umfasst somit zwei überlagerte Strahlenbündel mit unterschiedlich ausgerichteten Strahlachsen. Im Fall einer Kombination mit der Ausführungsvariante, in welcher die aufeinanderfolgenden Laserpulse abwechselnd dem ersten Strahlenbündel und dem zweiten Strahlenbündel zugeteilt werden, weist der Bearbeitungslaserstrahl aufeinanderfolgende Laserpulse auf, die jeweils eine um den betreffenden Winkel verschiedene Pulsrichtung respektive Projektionsachse oder Strahlachse aufweisen.

In einer Ausführungsvariante umfasst das Strahlteilsystem einen Strahlquerschnittteiler, der eingerichtet ist, einen ersten Querschnittsbereich des gepulsten Laserstrahls dem ersten Strahlteil zuzuführen, und einen zweiten Querschnittsbereich des gepulsten Laserstrahls dem zweiten Strahlteil zuzuführen, wobei sich der erste Querschnittsbereich und der zweite Querschnittsbereich nicht überlappen.

In einer Ausführungsvariante umfasst das Strahlteilsystem einen Strahlquerschnittvereiniger, der eingerichtet ist, den ersten Querschnittsbereich des gepulsten Laserstrahls einem ersten Querschnittsbereich des Bearbeitungslaserstrahls zuzuführen, und den zweiten Querschnittsbereich des gepulsten Laserstrahls einem zweiten Querschnittsbereich des Bearbeitungslaserstrahls zuzuführen, wobei sich der erste Querschnittsbereich des Bearbeitungslaserstrahls und der zweite Querschnittsbereich des Bearbeitungslaserstrahls nicht überschneiden.

In einer Ausführungsvariante ist das Scannersystem eingerichtet, den Bearbeitungslaserstrahl gemäss einem vorgegebenen Schnittlinien-Scannmuster ins Augengewebe zu lenken, wobei der erste Strahlteil eine erste Schnittlinie auf der ersten Aussenfläche des Lentikels erzeugt und der zweite Strahlteil eine zweite Schnittlinie auf der zweiten Aussenfläche des Lentikels erzeugt.

In einer Ausführungsvariante umfasst das Scannersystem ein erstes Scannermodul, das eingerichtet ist, den Bearbeitungslaserstrahl gemäss einer ersten Komponente des vorgegebenen Schnittlinien-Scannmusters in einer Vorschubrichtung entlang einer Bearbeitungslinie zu führen, und ein zweites Scannermodul, das eingerichtet ist, den Bearbeitungslaserstrahl gemäss einer der ersten Komponente überlagerten zweiten Komponente des vorgegebenen Schnittlinien-Scannmusters entlang einer quer zur Bearbeitungslinie verlaufenden Scannlinie zu lenken, mit einer wesentlich höheren Scanngeschwindigkeit im Vergleich zur Scanngeschwindigkeit des ersten Scannermoduls in Vorschubrichtung, wobei der erste Strahlteil eine erste Schnittlinie mit der ersten Komponente und der überlagerten zweiten Komponente auf der ersten Aussenfläche des Lentikels erzeugt und der zweite Strahlteil eine zweite Schnittlinie mit der ersten Komponente und der überlagerten zweiten Komponente auf der zweiten Aussenfläche des Lentikels erzeugt.

In einer Ausführungsvariante ist das Strahlteilsystem eingerichtet, den ersten Strahlteil und den zweiten Strahlteil derart zu erzeugen, dass der erste Strahlteil durch die Fokussieroptik auf die erste Aussenfläche fokussiert wird, die weiter von der Fokussieroptik entfernt ist als die zweite Aussenfläche, auf die der zweite Strahlteil durch die Fokussieroptik fokussiert wird.

In einer Ausführungsvariante umfasst die ophthalmologische Vorrichtung einen Intensitätsmodulator, der eingerichtet ist, die Lichtintensität des ersten Strahlteils im Verhältnis zur Lichtintensität des zweiten Strahlteils zu modulieren.

### Kurze Beschreibung der Zeichnungen

Nachfolgend wird eine Ausführung der vorliegenden Erfindung anhand eines Beispiels beschrieben. Das Beispiel der Ausführung wird durch die folgenden beigelegten Figuren illustriert:

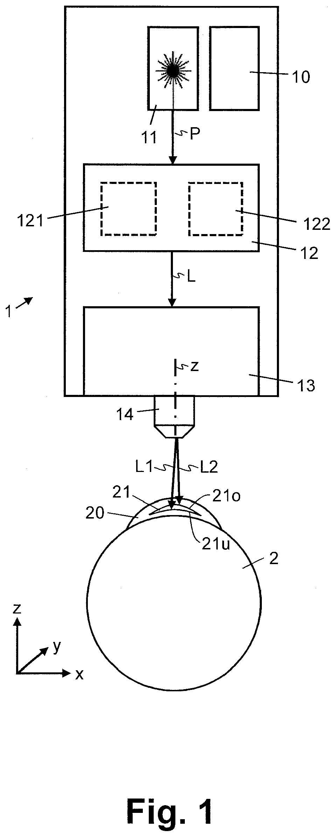

- Figur 1:: zeigt ein Blockdiagramm einer ophthalmologischen Vorrichtung zum Bearbeiten von Augengewebe mittels eines gepulsten Laserstrahls, wobei ein Bearbeitungslaserstrahl mit zwei Strahlteilen erzeugt wird, die auf verschiedene Aussenflächen eines im Augengewebe zu schneidenden Lentikels fokussiert sind, wie schematisch im Querschnitt dargestellt ist.

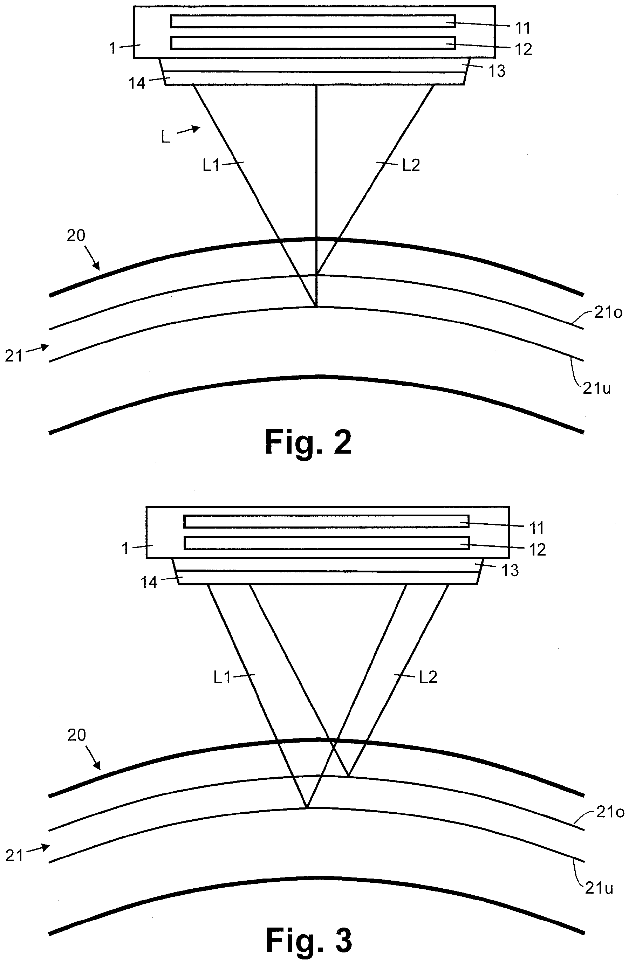

- Figur 2:: zeigt schematisch im Querschnitt den Bearbeitungslaserstrahl einer ophthalmologischen Vorrichtung, welcher zwei auf verschiedene Aussenflächen eines im Augengewebe zu schneidenden Lentikels fokussierte Strahlteile umfasst, die durch zwei sich nicht überlagernde Querschnittsbereiche des Bearbeitungslaserstrahls gebildet sind.
- Figur 3:: zeigt schematisch im Querschnitt den Bearbeitungslaserstrahl einer ophthalmologischen Vorrichtung, welcher zwei auf verschiedene Aussenflächen eines im Augengewebe zu schneidenden Lentikels fokussierte Strahlteile umfasst, die durch zwei Strahlenbündel des gepulsten Laserstrahls gebildet sind.

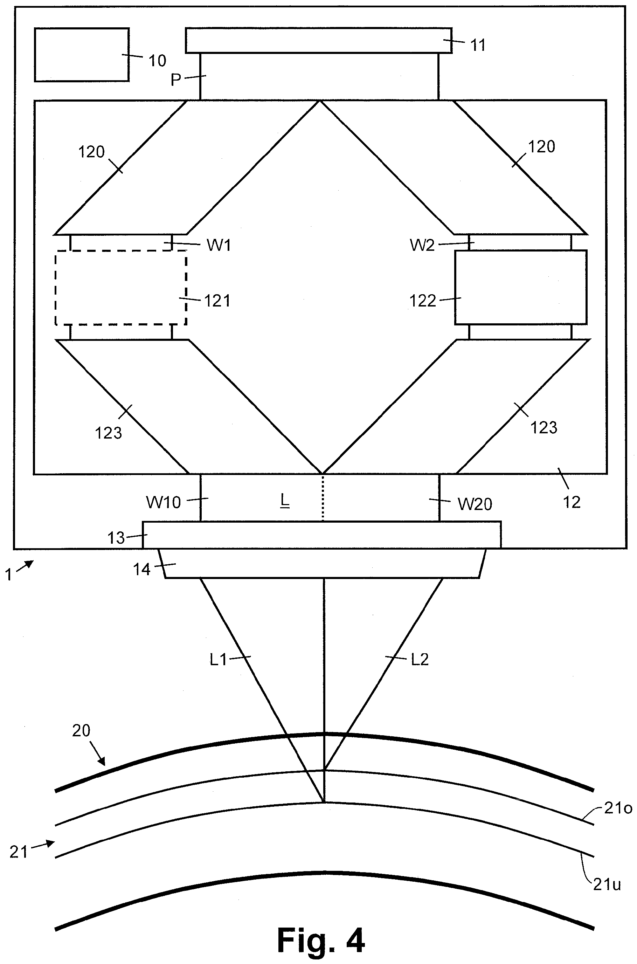

- Figur 4:: zeigt ein Blockdiagramm einer ophthalmologischen Vorrichtung zum Bearbeiten von Augengewebe mittels eines gepulsten Laserstrahls, welche einen Strahlquerschnittteiler, einen Divergenzmodulator und einen Strahlquerschnittvereiniger zur Erzeugung eines Bearbeitungslaserstrahls mit zwei Strahlteilen umfasst, die durch zwei sich nicht überlagernde Querschnittsbereiche des Bearbeitungslaserstrahls gebildet sind und auf verschiedene Aussenflächen eines im Augengewebe zu schneidenden Lentikels fokussiert sind, wie schematisch im Querschnitt dargestellt ist.

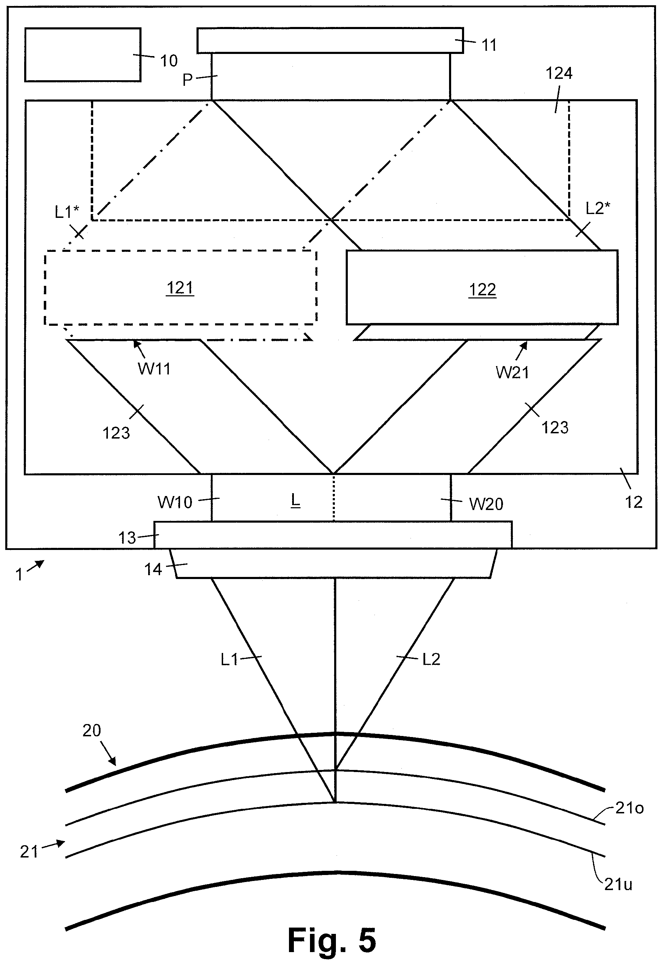

- Figur 5:: zeigt ein Blockdiagramm einer ophthalmologischen Vorrichtung zum Bearbeiten von Augengewebe mittels eines gepulsten Laserstrahls, welche einen Strahlteiler, einen Divergenzmodulator und einen Strahlquerschnittvereiniger zur Erzeugung eines Bearbeitungslaserstrahls mit zwei Strahlteilen umfasst, die durch zwei sich nicht überlagernde Querschnittsbereiche des Bearbeitungslaserstrahls gebildet sind und auf verschiedene Aussenflächen eines im Augengewebe zu schneidenden Lentikels fokussiert sind, wie schematisch im Querschnitt dargestellt ist.

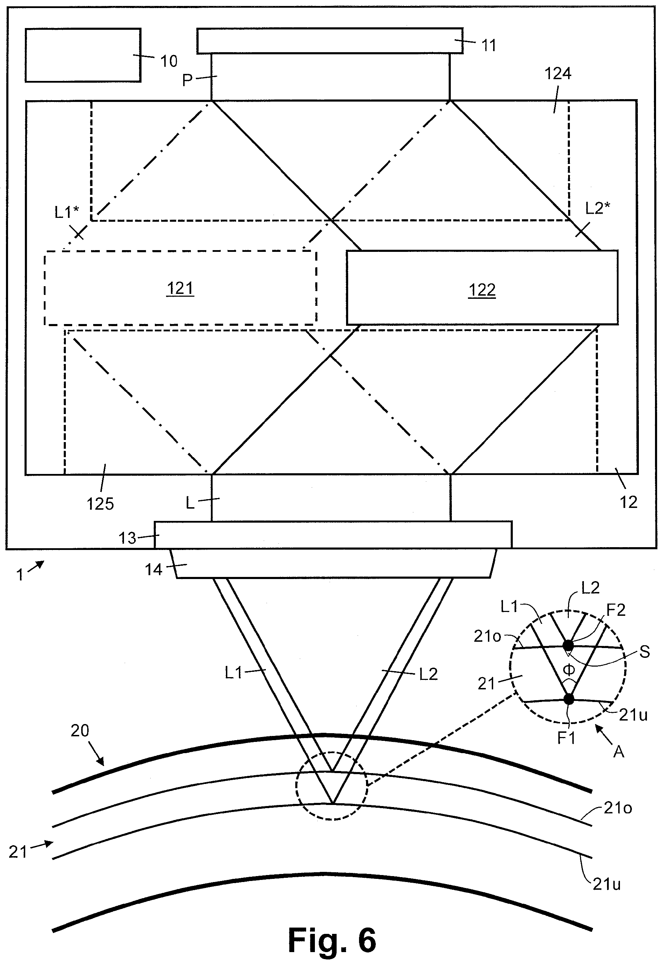

- Figur 6:: zeigt ein Blockdiagramm einer ophthalmologischen Vorrichtung zum Bearbeiten von Augengewebe mittels eines gepulsten Laserstrahls, welche einen Strahlteiler, einen Divergenzmodulator und einen Strahlvereiniger zur Erzeugung eines Bearbeitungslaserstrahls mit zwei koaxial verlaufenden Strahlteilen umfasst, die durch zwei Strahlenbündel mit alternierend zugeordneten Laserpulsen des gepulsten Laserstrahls gebildet sind und auf verschiedene Aussenflächen eines im Augengewebe zu schneidenden Lentikels fokussiert sind, wie schematisch im Querschnitt dargestellt ist.

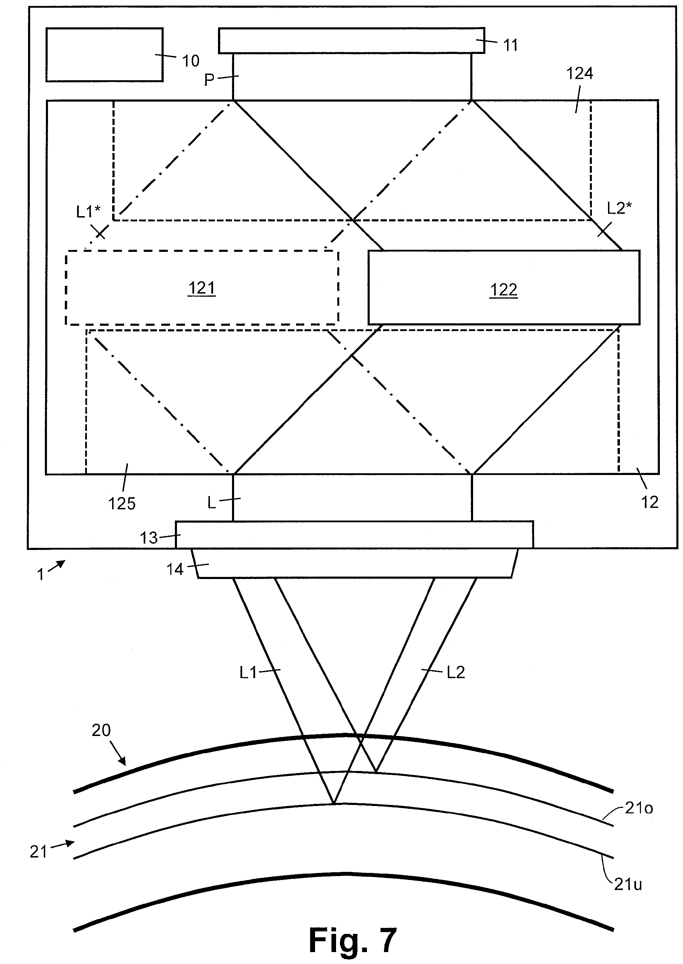

- Figur 7:: zeigt ein Blockdiagramm einer ophthalmologischen Vorrichtung zum Bearbeiten von Augengewebe mittels eines gepulsten Laserstrahls, welche einen Strahlteiler und einen Strahlvereiniger umfasst zur Erzeugung eines Bearbeitungslaserstrahls mit zwei Strahlteilen, die durch zwei mit einem Winkel zueinander verlaufenden Strahlenbündel des gepulsten Laserstrahls gebildet sind und auf verschiedene Aussenflächen eines im Augengewebe zu schneidenden Lentikels fokussiert sind, wie schematisch im Querschnitt dargestellt ist.

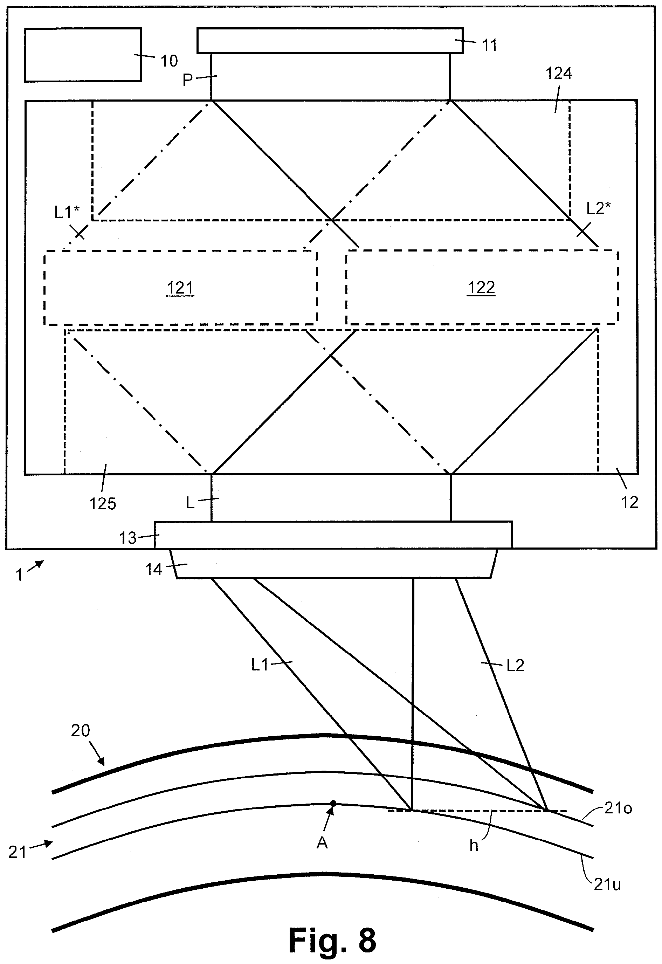

- Figur 8:: zeigt ein Blockdiagramm einer ophthalmologischen Vorrichtung zum Bearbeiten von Augengewebe mittels eines gepulsten Laserstrahls, welche einen Strahlteiler und einen Strahlvereiniger umfasst zur Erzeugung eines Bearbeitungslaserstrahls mit zwei Strahlteilen, die durch zwei mit einem Winkel zwischen ihren Strahlachsen verlaufenden Strahlenbündel des gepulsten Laserstrahls gebildet sind und fokussiert auf verschiedene Aussenflächen eines im Augengewebe zu schneidenden Lentikels gerichtet sind, wie schematisch im Querschnitt dargestellt ist.

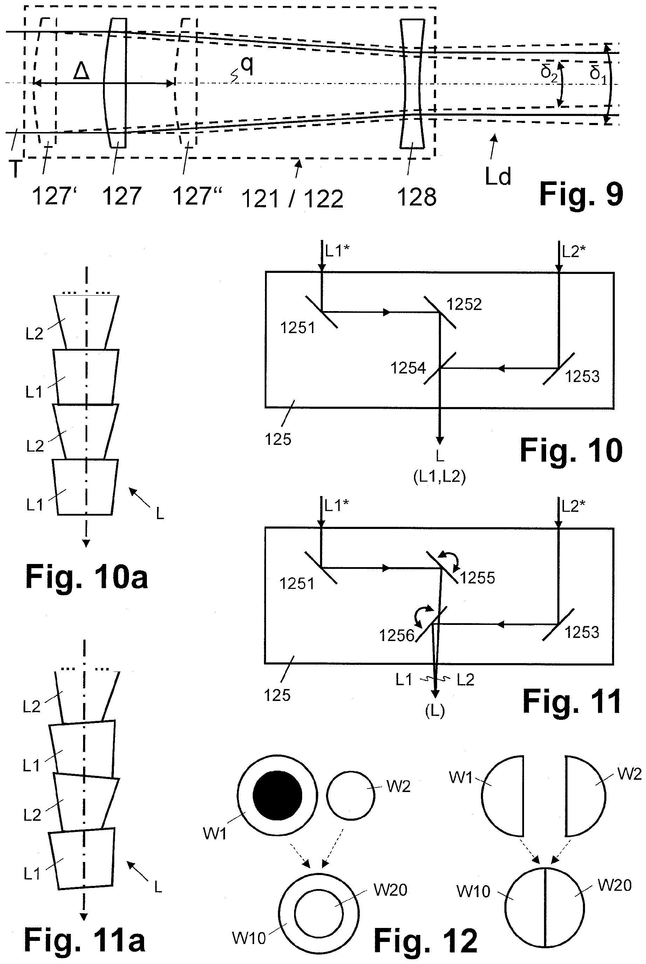

- Figur 9:: zeigt einen schematischen Querschnitt eines Abschnitts des Strahlengangs in einem Divergenzmodulator mit mindestens einer verschiebbaren Linse und illustriert die durch die Verschiebung der Linse veränderte Divergenz des Laserstrahls.
- Figur 10:: zeigt ein Blockdiagramm eines Strahlvereinigers mit optischen Elementen zum koaxialen Vereinen von zwei Strahlenbündeln auf einen Bearbeitungslaserstrahl.
- Figur 10a:: illustriert schematisch im Querschnitt einen Bearbeitungslaserstrahl mit zwei Strahlteilen, welche aus zwei koaxial vereinigten Strahlenbündeln mit unterschiedlicher Divergenz erzeugt wurden.
- Figur 11:: zeigt ein Blockdiagramm eines Strahlvereinigers mit optischen Elementen zum Vereinen von zwei Strahlenbündeln auf einen Bearbeitungslaserstrahl mit einem Winkel zwischen den Strahlachsen der Strahlenbündel.
- Figur 11a:: illustriert schematisch im Querschnitt einen Bearbeitungslaserstrahl mit zwei Strahlteilen, welche aus zwei Strahlenbündeln erzeugt wurden, die einen Winkel zwischen ihren Strahlachsen und eine unterschiedliche Divergenz aufweisen.
- Figur 12:: illustriert Beispiele von sich nicht überlappenden Strahlquerschnitten des gepulsten Laserstrahls, die sich nicht überlappen; links konzentrisch angeordnete Strahlquerschnitte, rechts nebeneinander angeordnete Strahlquerschnitte.

### Wege zur Ausführung der Erfindung

In den Figuren 1-8 bezieht sich das Bezugszeichen 1 jeweils auf eine ophthalmologische Vorrichtung zum Bearbeiten von Augengewebe 20 mittels Laserpulsen. Ein Auge 2 ist schematisch im Querschnitt in Figur 1 illustriert. Dabei bezieht sich das Bezugszeichen 20 auf zu bearbeitendes Augengewebe, insbesondere die Cornea. Wie in den Figuren 1-8 schematisch dargestellt ist, ist die ophthalmologische Vorrichtung 1 eingerichtet im Augengewebe 20, insbesondere in der Cornea, ein Lentikel 21 zu schneiden, das eine obere Aussenfläche 21o und eine untere Aussenfläche 21u aufweist. Die obere Aussenfläche 21o ist der externen Oberfläche der Cornea 20 respektive der ophthalmologischen Vorrichtung 1 zugewandt. Die untere Aussenfläche 21u ist von der externen Oberfläche der Cornea 20 respektive der ophthalmologischen Vorrichtung 1 abgewandt.

Wie in den Figuren 1-8 schematisch illustriert ist, umfasst die ophthalmologische Vorrichtung 1 eine Laserquelle 11. Die Laserquelle 11 ist eingerichtet einen gepulsten Laserstrahl P zu erzeugen. In einer Ausführungsvariante ist die Laserquelle 11 eingerichtet einen Laserstrahl P mit Femtosekundenlaserpulsen zu erzeugen.

Die ophthalmologische Vorrichtung 1 umfasst überdies ein Strahlteilsystem 12, das eingerichtet ist, aus dem gepulsten Laserstrahl P einen Bearbeitungslaserstrahl L zu erzeugen, welcher zwei Strahlteile L1, L2 umfasst wie nachfolgend beschrieben wird.

Die ophthalmologische Vorrichtung 1 umfasst zudem ein Scannersystem 13, dem das Strahlteilsystem 12 vorgeschaltet ist, und eine Fokussieroptik 14. Der vom Strahlteilsystem 12 erzeugte Bearbeitungslaserstrahl L wird zur Bearbeitung des Augengewebes 20 dem Scannersystem 13 zugeführt. Das Scannersystem 13 ist eingerichtet, den Bearbeitungslaserstrahl L gemäss einem vorgegebenen x/y-Scannmuster über die Fokussieroptik 14 ins Augengewebe 20 zu lenken, um im Augengewebe 20 Schnitte auszuführen. In einer Ausführungsvariante umfasst das Scannersystem 13 als Scannermodul ein Antriebssystem, das mit der Fokussieroptik 14 gekoppelt ist und eingerichtet ist, die Fokussieroptik 14 in x/y-Bearbeitungsrichtungen einer Bearbeitungsebene zu verfahren, welche normal zu einer optischen Achse y der Fokussieroptik 14 angeordnet ist. In einer Ausführungsvariante umfasst das Scannersystem 13 als Alternative oder zusätzlich zum Antriebssystem eines oder mehrere strahlablenkende x/y-Scannermodule, z.B. Scannermodule mit beweglichen Spiegeln, die um eine oder zwei Scannachsen schwenkbar sind, beispielsweise Galvano-, Piezo-, Polygon- oder Resonanz-Scannermodule, oder AOM- (Acusto-optischer Modulator) oder EOM- Scannermodul (Electro-optischer Modulator). In einer Ausführungsvariante ist ein Scannermodul des Scannersystems 13 als Slow-Scanner eingerichtet, den Bearbeitungslaserstrahl L gemäss einer ersten Komponente des vorgegebenen Schnittlinien-Scannmusters in einer Vorschubrichtung entlang einer Bearbeitungslinie zu führen, und ein weiteres Scannermodul des Scannersystems 13 ist als Fast-Scanner eingerichtet, den Bearbeitungslaserstrahl L gemäss einer der ersten Komponente überlagerten zweiten Komponente des vorgegebenen Schnittlinien-Scannmusters entlang einer quer zur Bearbeitungslinie verlaufenden Scannlinie zu lenken, mit einer vergleichsweise wesentlich höheren Scanngeschwindigkeit im Vergleich zur Scanngeschwindigkeit des Slow-Scanners in Vorschubrichtung.

Die Fokussieroptik 14 ist eingerichtet, den Bearbeitungslaserstrahl L ins Augengewebe 20 zu fokussieren. Die Fokussieroptik 14 umfasst eine oder mehrere optische Linsen. Zur Einstellung der Brennweite (Fokus) umfasst die Fokussieroptik 14 mindestens eine bewegliche Linse. Alternativ kann die gesamte Fokussieroptik 14 verschoben werden. In einer Ausführungsvariante umfasst die Fokussieroptik 14 zudem einen oder mehrere Antriebe, z.B. Elektromotoren, zur automatisierten Bewegung der beweglichen Linse(n) respektive der gesamtes Fokussieroptik 14 und der dadurch bewirkten Einstellung und Anpassung des Fokus respektive der durch das Scannersystem 13 abtast- und bearbeitbaren Fokalfläche(n).

Wie in den Figuren 1-8 schematisch dargestellt ist, umfasst die ophthalmologische Vorrichtung 1 einen Schaltkreis 10 zur Steuerung der Laserquelle 11, des Scannersystems 13, und der Fokussieroptik 14 sowie von nachfolgend beschriebenen Divergenzmodulatoren 121, 122, Strahlteilern 124, Strahlvereiniger 125 und Intensitätsmodulator(en). Der Schaltkreis 10 realisiert eine programmierbare Steuervorrichtung und umfasst beispielsweise ein oder mehrere Prozessoren mit Programm- und Datenspeicher sowie programmierte Softwaremodule zur Steuerung der Prozessoren, und/oder andere programmierbare Schaltungen oder Logikeinheiten wie ASICs (Application Specific Integrated Circuit). Zur Steuerung der verschiedenen Komponenten und Module der ophthalmologischen Vorrichtung 1 erzeugt der Schaltkreis 10 entsprechende Steuersignale für diese Komponenten und Module.

In einer Variante umfasst die ophthalmologische Vorrichtung 1 einen (zum bereits vorhandenen Intensitätsmodulator der Laserquelle 11 zusätzlichen) Intensitätsmodulator, der eingerichtet ist, die Lichtintensität des ersten Strahlteils L1 im Verhältnis zur Lichtintensität des zweiten Strahlteils L2 zu modulieren. In einer Ausführungsvariante ist der Intensitätsmodulator dadurch ausgeführt, dass der Schaltkreis 10 die Intensität respektive Leistung der Laserquelle 11 synchronisiert mit der Erzeugung der Strahlteile L1, L2 steuert und moduliert. In einer im Strahlengang eines der Strahlteile L1, L2 implementierten Ausführungsvariante umfasst der Intensitätsmodulator einen Polarisationsrotator (z.B. Lambda/2-Platten) und/oder Polarisationsstrahlteiler. In einer Ausführungsvariante ist der Intensitätsmodulator im Strahlvereiniger 125 implementiert, wobei polarisationsempfindliche Beschichtungen die Aufgabe der Intensitätsmodulation übernehmen, indem nur ein bestimmter Intensitätsanteil der Strahlung auf die gemeinsame Strahlachse eingekoppelt wird.

Wie obenstehend angeführt, ist das Strahlteilsystem 12 eingerichtet, aus dem gepulsten Laserstrahl P einen Bearbeitungslaserstrahl L zu erzeugen, der zwei Strahlteile L1, L2 umfasst. Wie in den Figuren 1-7 illustriert ist, weisen die Strahlteile L1 und L2 eine unterschiedliche Divergenz auf, so dass sie durch die Fokussieroptik 14 auf unterschiedliche Fokaltiefen respektive Brennweiten fokussiert werden, ohne dass dabei eine Verstellung der Fokussieroptik 14 durchgeführt wird respektive durchgeführt werden muss. In den Ausführungsvarianten gemäss den Figuren 2, 4 und 5 sind die Strahlteile L1 und L2 verschiedene, sich nicht überlappende (örtlich getrennte) Strahlquerschnitte W10, W20 des Bearbeitungslaserstrahls L. In den Ausführungsvarianten gemäss den Figuren 3, 6-8, 10 und 11 sind die Strahlteile L1 und L2 des Bearbeitungslaserstrahls L aus zwei verschiedenen Strahlenbündeln L1*, L2* erzeugt. In der Ausführungsvariante gemäss der Figur 6 sind die Laserpulse der Strahlteile L1 und L2 zeitlich zueinander verschoben, derart dass im Bearbeitungslaserstrahl L abwechselnd jeweils ein Laserpuls von einem der beiden Strahlteile L1, das von einem der Strahlenbündel L1* erzeugt wird, auf den nächsten Laserpuls des anderen der Strahlteile L2 folgt, der vom anderen Strahlenbündel L2* erzeugt wird, wie nachfolgend beschrieben und in der Figur 10a schematisch dargestellt wird. In den Ausführungsvarianten gemäss den Figuren 3, 7, 8, 10 und 11 sind die Strahlteile L1 und L2 des Bearbeitungslaserstrahls L aus zwei verschiedenen Strahlenbündeln L1*, L2* erzeugt, die durch Strahlteilung aus dem gepulsten Laserstrahl P erzeugt werden, wobei je nach Variante eine Aufteilung der Strahlenergie des gepulsten Laserstrahls P auf die Strahlenbündel L1*, L2* erfolgt oder, wie gemäss der Ausführungsvariante der Figur 6, die Laserpulse des gepulsten Laserstrahls P abwechselnd den beiden Strahlenbündeln L1*, L2* zugeschaltet werden. In der Ausführungsvariante der Figur 4 umfasst das Strahlteilsystem 12 einen Strahlquerschnittteiler 120, der eingerichtet ist, einen ersten Querschnittsbereich W1 des gepulsten Laserstrahls P und einen zweiten Querschnittsbereich W2 des gepulsten Laserstrahls P, die sich nicht überlappen, zu trennen. Figur 12 illustriert zwei Beispiele von sich nicht überlappenden Strahlquerschnitten W1, W2 des gepulsten Laserstrahls P (im oberen Teil); links konzentrisch angeordnete Strahlquerschnitte W1, W2; rechts nebeneinander angeordnete Strahlquerschnitte W1, W2, wobei die entsprechenden wiedervereinten, sich nicht überlappenden Strahlquerschnitte W10, W20 jeweils im unteren Teil der Figur 12 illustriert sind. In anderen Varianten ist eine segmentförmige Aufteilung des Strahlquerschnitts vorgesehen, z.B. in Form von Quadranten oder Sextanten. Der Strahlquerschnittteiler 120 ist z.B. ein Reflexionsstrahlteiler, der den Strahlquerschnitt des gepulsten Laserstrahls P aufteilt. Der Strahlquerschnittteiler 120 umfasst z.B. einen Strahlteilerwürfel, der mittels reflektierenden Beschichtungen und/oder Masken den ersten Querschnittsbereich W1 und den zweiten Querschnittsbereich W2 des gepulsten Laserstrahls P in unterschiedliche Richtungen ablenkt respektive durchlässt. Wie in der Figur 4 schematisch dargestellt ist, umfasst das Strahlteilsystem 12 zudem mindestens einen Divergenzmodulator 121, 122, der eingerichtet ist, die Divergenz des ihm zugeführten Laserstrahlteils dynamisch zu verändern. In einer Variante mit *einem* Divergenzmodulator 122 führt der Strahlquerschnittteiler 120 den entsprechenden Querschnittsbereich W2 des gepulsten Laserstrahls P über den Divergenzmodulator 122 zu einem Strahlvereiniger respektive einem Strahlquerschnittvereiniger 123 des Strahlteilsystems 12, wohingegen der Querschnittsbereich W1 des gepulsten Laserstrahls P ohne Divergenzmodulation direkt dem Strahlvereiniger respektive Strahlquerschnittvereiniger 123 zugeführt wird. Bei einer Variante mit *zwei* Divergenzmodulatoren 121, 122 führt der Strahlquerschnittteiler 120 die Querschnittsbereiche W1 und W2 des gepulsten Laserstrahls P über den jeweils zugeordneten Divergenzmodulator 121, 122 zum Strahlvereiniger respektive Strahlquerschnittvereiniger 123 des Strahlteilsystems 12. Der Strahlquerschnittvereiniger 123 umfasst z.B. einen Strahlteilerwürfel, der mit reflektierenden Beschichtungen und/oder Masken versehen ist, und eingerichtet ist, die vom Strahlquerschnittteiler 120 direkt oder über den Divergenzmodulator 121, 122 zugeführten Querschnittsbereiche W1 und W2 des gepulsten Laserstrahls P einem ersten Querschnittsbereich W10 und einem zweiten Querschnittsbereich W20 des Bearbeitungslaserstrahls L zuzuführen, wobei sich die beiden Querschnittsbereiche W10, W20 des Bearbeitungslaserstrahls L nicht überschneiden. Durch die Strahlquerschnittteilung und die Strahlwiedervereinigung respektive Strahlquerschnittwiedervereiningung der beiden unterschiedlich divergenzmodulierten Strahlengänge wird eine Abschattung und eine dadurch bewirkte Beeinflussung der beiden erzeugten Foki der Strahlteile L1, L2 vermieden. Eine Kombination von Strahlquerschnittsteiler und Strahlquerschnittvereiniger ist nicht zwingend erforderlich. Eines der beiden Elemente lässt sich durch einen normalen Strahlteiler ersetzen, wenn gewährleistet ist, dass das andere Element eine Separierung der Querschnittsbereiche W10 und W20 ausreichend genau bewirkt.

Die Figur 9 illustriert schematisch eine Ausführungsvariante des Divergenzmodulators 121, 122 mit zwei seriell angeordneten optischen Linsen 127, 128, von denen mindestens eine zur Modulation der Divergenz des zugeführten Laserstrahlteils T auf einer optischen Übertragungsachse q verschiebbar ist. Zur dynamischen Modulation der Divergenz des zugeführten Laserstrahlteils T ist die bewegliche Linse 127 mit einem Bewegungstreiber gekoppelt. Wie im Beispiel der Figur 9 ersichtlich ist, weist der zugeführte Laserstrahlteil T bei einer ersten Grundstellung 127' der beweglichen Linse eine entsprechende Divergenz δ₂ auf. Bei einer Verschiebung der beweglichen Linse 127 auf der Übertragungsachse w verändert sich die Divergenz des zugeführten Laserstrahlteils T kontinuierlich und hat bei der um die Auslenkungsdistanz Δ verschobenen Stellung 1 27" eine veränderte Divergenz δ₁.

Der durch das Strahlteilsystem 12 aus dem gepulsten Laserstrahl P erzeugte Bearbeitungslaserstrahl L mit den beiden Querschnittsbereichen W10, W20 wird dem Scannersystem 13 zum Schneiden der oberen und unteren Aussenflächen 21u, 21o des Lentikels 21 im Augengewebe 20 zugeführt.

Bei der Variante mit *einem* Divergenzmodulator 122 steuert der Schaltkreis 10 die Fokussieroptik 14 synchronisiert mit dem Scannersystem 13 so, dass der durch den nicht divergenzmodulierten Querschnittsbereich W1 respektive W10 gebildete Strahlteil L1 des Bearbeitungslaserstrahls L auf die untere Aussenfläche 21u des zu schneidenden Lentikels 21 fokussiert wird. Gleichzeitig und synchronisiert steuert der Schaltkreis 10 den Divergenzmodulator 122 so, dass der durch den divergenzmodulierten Querschnittsbereich W2 respektive W20 gebildete Strahlteil L2 des Bearbeitungslaserstrahls L, bei gleichbleibender Einstellung der Fokussieroptik 14, auf die obere Aussenfläche 21o des zu schneidenden Lentikels 21 fokussiert wird.

Bei der Variante mit *zwei* Divergenzmodulatoren 121, 122 steuert der Schaltkreis 10 die Fokussieroptik 14 so, dass eine feste Schnittposition respektive Schnitttiefe eingestellt wird (nach erfolgter Einstellung kann die Fokussieroptik 14 fixiert bleiben und muss nicht weiter verschoben werden). Der Schaltkreis 10 steuert den Divergenzmodulator 121 synchronisiert mit dem Scannersystem 13 so, dass der durch den divergenzmodulierten Querschnittsbereich W1 respektive W10 gebildete Strahlteil L1 des Bearbeitungslaserstrahls L, bei festbleibender Einstellung der Fokussieroptik 14, auf die untere Aussenfläche 21u des zu schneidenden Lentikels 21 fokussiert wird. Gleichzeitig und synchronisiert steuert der Schaltkreis 10 den Divergenzmodulator 122 so, dass der durch den divergenzmodulierten Querschnittsbereich W2 respektive W20 gebildete Strahlteil L2 des Bearbeitungslaserstrahls L, bei festbleibender Einstellung der Fokussieroptik 14, auf die obere Aussenfläche 21o des zu schneidenden Lentikels 21 fokussiert wird.

Das Scannersystem 13 lenkt den Bearbeitungslaserstrahl L hinsichtlich der x/y-Positionierung zur Bearbeitung des Augengewebes 20. Für jeden durch das Scannersystem 13 gemäss einem definierten Scannmuster bestimmten x/y-Bearbeitungspunkt steuert der Schaltkreis 10 die Divergenzmodulation der Divergenzmodulatoren 121, 122, so dass mittels des Strahlteils L1 eine fokussierte Bearbeitung des Augengewebes 20 auf der unteren Aussenfläche 21u und mittels des Strahlteils L2 eine fokussierte Bearbeitung des Augengewebes 20 auf der oberen Aussenfläche 21o des Lentikels erfolgt, ohne dass dazu eine Änderung der Fokussierung respektive Fokussiervorrichtung 14 vorgenommen wird respektive werden muss. Je nach Fokussierbereich und/oder technischer Umsetzung kann bei zwei Divergenzmodulatoren auch auf eine Einstellung der Fokussiervorrichtung gänzlich verzichtet werden

Die Ausführungsvariante der Figur 5 unterscheidet sich von derjenigen gemäss Figur 4 durch ein anders ausgestaltetes Strahlteilsystem 12 und eine entsprechend angepasste Steuerung durch den Schaltkreis 10. Das Strahlteilsystem 12 gemäss Figur 5 umfasst einen Strahlteiler 124, der eingerichtet ist, den gepulsten Laserstrahl P in zwei Strahlenbündel L1* und L2* zu teilen. Wie in der Ausführungsvariante der Figur 4 umfasst das Strahlteilsystem 12 einen oder zwei Divergenzmodulatoren 121, 122, über welche der Strahlteiler 124 die Strahlenbündel L1^{∗} und L2* führt. In der Variante mit *einem* Divergenzmodulator 122 führt der Strahlteiler 124 das entsprechende Strahlenbündel L2* des gepulsten Laserstrahls P über den Divergenzmodulator 122 zu einem Strahlvereiniger respektive Strahlquerschnittvereiniger 123 des Strahlteilsystems 12, wohingegen das Strahlenbündel L1^{∗} des gepulsten Laserstrahls P ohne Divergenzmodulation direkt dem Strahlvereiniger respektive Strahlquerschnittvereiniger 123 zugeführt wird. Bei der Variante mit *zwei* Divergenzmodulatoren 121, 122 führt der Strahlteiler 124 die Strahlenbündel L1^{∗} und L2* des gepulsten Laserstrahls P über den jeweils zugeordneten Divergenzmodulator 121, 122 zum Strahlvereiniger respektive Strahlquerschnittvereiniger 123 des Strahlteilsystems 12.

In der Ausführungsvariante der Figur 6 (optional auch in den Ausführungsvarianten der Figuren 7 und 8) ist der Strahlteiler 124 eingerichtet, zur Teilung des gepulsten Laserstrahls P in zwei Strahlenbündel L1*, L2*, aufeinanderfolgende Laserpulse des gepulsten Laserstrahls P abwechselnd entweder dem ersten Strahlenbündel L1^{∗} oder dem zweiten Strahlenbündel L2* zuzuführen. Der Schaltkreis 10 synchronisiert den Strahlteiler 124 derart mit der Laserquelle 11 und dem Scannersystem 13, dass die Reihenfolge so erfolgt, dass von den beiden aufeinanderfolgenden Laserpulsen, die vom Scannersystem 13 ohne veränderte Ablenkung des Bearbeitungsstrahls L ins Augengewebe 20 gelenkt werden, der erste Laserpuls dem ersten Strahlenbündel L1* respektive Strahlteil L1 zugeteilt wird, der auf die untere Aussenfläche 21u fokussiert wird, und dass der unmittelbar darauf folgende zweite Laserpuls dem zweiten Strahlenbündel L2* respektive Strahlteil L2 zugeteilt wird, der auf die obere Aussenfläche 21o fokussiert wird. Durch die abwechselnde Pulsfolge, ein Laserpuls fokussiert zuerst auf die untere Aussenfläche 21u gefolgt von einem Laserpuls fokussiert auf die obere Aussenfläche 21o, kann eine Abschattung und eine dadurch bewirkte Beeinflussung der beiden erzeugten Foki der Strahlteile L1, L2 vermieden werden, ohne dass dazu unterschiedliche Strahlwinkel der beiden Strahlteile L1, L2 erforderlich sind.

In einer Ausführungsvariante umfasst der Strahlteiler 124 einen als (schneller) optischer Schalter ausgeführten optischen Modulator, z.B. AOM oder EOM, der synchronisiert mit der Laserquelle 11 gekoppelt ist und eingerichtet ist, den ersten Puls des gepulsten Laserstrahls P dem ersten Strahlenbündel L1^{∗} zuzuführen, und den zweiten Puls des gepulsten Laserstrahls P dem zweiten Strahlenbündel L2* zuzuführen.

In einer alternativen Ausführungsvariante umfasst der Strahlteiler 124 einen optischen Strahlteiler, der eingerichtet ist, den gepulsten Laserstrahl P in das erste Strahlenbündel L1* und in das zweite Strahlenbündel L2* aufzuteilen. Der Strahlteiler 124 umfasst überdies zwei als (schnelle) optische Schalter ausgeführte optische Modulatoren, z.B. AOM oder EOM, die synchronisiert mit der Laserquelle 11 gekoppelt im ersten Strahlenbündel L1* respektive im zweiten Strahlenbündel L2* angeordnet sind. Der erste optische Modulator ist eingerichtet, im ersten Strahlenbündel L 1 * den zweiten Puls des gepulsten Laserstrahls P auszublenden. Der zweite optische Modulator ist eingerichtet, im zweiten Strahlenbündel L2* den ersten Puls des gepulsten Laserstrahls P auszublenden.

In einer Ausführungsvariante umfassen die optischen Modulatoren EOM-Module, die eingerichtet sind, die Polarisation des einfallenden gepulsten Laserstrahl P zu modulieren, und mit polarisationsempfindlichen Strahlteilern und/oder Polarisationsfiltern kombiniert sind, um die aufgeteilten Strahlenbündel L1* und L2* zu erzeugen.

Wie in der Figur 6 im vergrösserten Ausschnitt A schematisch durch den Schattenkegel S illustriert ist, wird im Fokus F2 des auf die obere Aussenfläche 21o des Lentikels 21 fokussierten Strahlteils L2 durch die Wechselwirkung eines eingestrahlten Laserpulses mit dem Augengewebe eine Abschattung des darunterliegenden Augengewebes verursacht. Durch diese Abschattung wird die eingestrahlte Energie im Fokus F1 des Strahlteils L1 auf der unteren Aussenfläche 21u des Lentikels 21 reduziert. Bei kurzen Laserpulsen, beispielsweise Femtosekundenlaserpulse (fs), z.B. 100fs, ist diese Beeinträchtigung relativ gering und es kann auf die oben beschriebenen Pulsmodulation verzichtet werden, da durch die kurze Pulsdauer eines einzelnen Laserpulses die Entstehung von störenden (grossen) Blasen erst zeitverzögert erfolgt. Bei grossen Fokuswinkeln Φ, z.B. Φ>30°, und einem gewissen Mindestabstand zwischen der oberen und unteren Aussenfläche 21o, 21u des Lentikels 21, z.B. grösser als 20µm bei einem Wechselwirkungsbereich von ca. 2µm um den Fokus F2, also ein Wechselwirkungsbereich kleiner als ca. 10% der Dicke des Lentikels 21, ist diese Reduktion der Strahlenergie im Fokus F1 auf der unteren Aussenfläche 21u des Lentikels 21 gering und kann durch eine entsprechende Erhöhung der Intensität des Strahlteils L1 respektive des Strahlenbündels L1^{∗} kompensiert werden. Dazu ist der Intensitätsmodulator (respektive der Schaltkreis 10 zur Steuerung der Laserquelle 11) eingerichtet ist, die Lichtintensität des ersten Strahlteils L1 respektive des ersten Strahlenbündels L 1 * (im Verhältnis zur Lichtintensität des zweiten Strahlteils L2 respektive des zweiten Strahlenbündels L2*) entsprechend zu erhöhen, um die Abschattung durch den Schattenkegel S (respektive die dadurch verminderte Strahlenergie auf der unteren Aussenfläche 21u des Lentikels 21) zu kompensieren, der durch die Wechselwirkung von Laserpulsen im Fokus F2 auf der oberen Aussenfläche 21o des Lentikels 21 verursacht wird.

Die Ausführungsvarianten gemäss den Figuren 6, 7 und 8 unterscheiden sich von denjenigen der Figuren 4 und 5 durch ein anders ausgestaltetes Strahlteilsystem 12 und eine entsprechend angepasste Steuerung durch den Schaltkreis 10. Das Strahlteilsystem 12 in der Ausführungsvariante gemäss den Figuren 6, 7 und 8 umfasst, wie diejenige der Figur 5, einen Strahlteiler 124 und einen oder zwei Divergenzmodulatoren 121, 122, über welche der Strahlteiler 124 die Strahlenbündel L1^{∗} und L2* dem Strahlvereiniger 125 zuführt. Im Unterschied zu der Ausführungsvariante der Figur 5 ist der Strahlvereiniger 125 jedoch nicht als Strahlquerschnittvereiniger 123 ausgeführt, sondern als Strahlvereiniger, der eingerichtet ist, die vom Strahlteiler 124 direkt oder über den Divergenzmodulator 121, 122 zugeführten Strahlenbündel L1* und L2* des gepulsten Laserstrahls P auf den Bearbeitungslaserstrahl L zu vereinen. Je nach Ausführungsvariante werden die beiden Strahlenbündel L1*, L2* auf Strahlteile L1, L2 mit einer gemeinsamen Strahlachse (Figuren 6 und 10) oder mit einem Winkel zwischen einer Strahlachse des ersten Strahlenbündels L1* und einer Strahlachse des zweiten Strahlenbündels L2* (Figuren 7, 8 und 11) zum Bearbeitungslaserstrahl L vereint. Durch die Vereinigung der beiden Strahlenbündel L1*, L2* auf Strahlteile L1, L2 mit einem Winkel zwischen ihren Strahlachsen (Figuren 7 und 8) wird eine Abschattung und dadurch bewirkte Beeinflussung der beiden erzeugten Foki der Strahlteile L1, L2 vermieden, ohne dass dazu die Laserpulse (wie in der Variante gemäss Figur 6) alternierend den beiden Strahlenbündel L1*, L2* zugeschaltet werden müssen. Um die Fokustiefe insgesamt einzustellen, umfasst die ophthalmologische Vorrichtung 1 in diesen Ausführungsvarianten, zusätzlich oder als Alternative zu der verschiebbaren respektive einstellbaren Fokussieroptik 14, ein Zoomsystem zur Einstellung der Fokustiefe auf einem oder beiden der Strahlengänge L1*, L2*, das vor oder nach dem einen oder den beiden Divergenzmodulatoren 121, 122 angeordnet ist. Je nach Fokussierbereich und/oder technischer Umsetzung kann bei zwei Divergenzmodulatoren auch auf eine Einstellung der Fokussieroptik respektive Zoomsysteme gänzlich verzichtet werden.

Figur 10 illustriert eine Ausführungsvariante des Strahlvereinigers 125 mit Umlenkspiegeln 1251 und 1252 zum Einkoppeln des Strahlenbündels L1^{∗} auf den Strahlengang respektive die Strahlachse des Bearbeitungslaserstrahls L. Der Strahlvereiniger 125 umfasst einen weiteren Umlenkspiegel 1253 und einen halbdurchlässigen Spiegel 1254 zum koaxialen Einkoppeln des Strahlenbündels L2* auf den Strahlengang respektive die Strahlachse des Bearbeitungslaserstrahls L. Der Fachmann wird verstehen, dass die beiden Strahlenbündel L1^{∗} und L2* auch über einen Strahlteilerwürfel vereint werden können. Die beiden Strahlenbündel L1^{∗} und L2* verlaufen koaxial als Strahlteile L1 und L2 auf der Strahlachse des Bearbeitungslaserstrahls L, jeweils abwechselnd mit einem Laserpuls vom ersten Strahlenbündel L1* mit einer ersten Divergenz und einem Laserpuls vom zweiten Strahlenbündel L2* mit einer zweiten Divergenz, wie in Figur 10a schematisch dargestellt ist und mit Bezug zur Figur 6 oben beschrieben wurde.

Die Ausführungsvariante gemäss Figur 11 unterscheidet sich von derjenigen der Figur 10 dadurch, dass der Strahlvereiniger 125 einen Spiegel 1255 und einen halbdurchlässigen Spiegel 1256 zum Einkoppeln der Strahlenbündel L1*, L2* auf den Strahlengang des Bearbeitungslaserstrahls L umfasst, von welchen mindestens einer verdrehbar ausgestaltet ist, derart, dass die Strahlenbündel L1^{∗} und L2* mit einem Winkel zur Strahlachse des Bearbeitungslaserstrahls L respektive zwischen den Strahlachsen der Strahlenbündel L1*, L2* in den Strahlengang des Bearbeitungslaserstrahls L eingekoppelt werden können, wie oben mit Bezug zu den Figuren 7 und 8 beschrieben wurde. Der erzeugte Bearbeitungslaserstrahl L umfasst in unterschiedlichen Richtungen verlaufende Strahlteile L1, L2. In der Ausführungsvariante gemäss Figur 7 unterscheiden sich die aufeinanderfolgenden Strahlteile L1, L2 zudem auch in ihrer Divergenz. In der Ausführungsvariante gemäss Figur 8 haben die aufeinanderfolgenden Strahlteile L1, L2 dieselbe Divergenz wie nachfolgend detaillierter beschrieben wird. In einer Variante, in der die Strahlvereinigung mit unterschiedlicher Strahlrichtung der Strahlteile L1, L2 mit der Strahlteilung mit alternierender Pulszuschaltung zum ersten respektive zweiten Strahlenbündel L1*, L2* kombiniert wird, weist der Bearbeitungslaserstrahl L in unterschiedlichen Richtungen verlaufende Strahlteile L1, L2 auf, jeweils abwechselnd mit einem Laserpuls vom ersten Strahlenbündel L1* mit einer ersten Strahlachsenrichtung und einem Laserpuls vom zweiten Strahlenbündel L2^{∗} mit einer davon verschiedenen zweiten Strahlachsenrichtung, wie in Figur 11a schematisch dargestellt ist.

Das Scannersystem 13 lenkt den Bearbeitungslaserstrahl L hinsichtlich der x/y-Positionierung zur Bearbeitung des Augengewebes 20. Für jeden durch das Scannersystem 13 gemäss einem definierten Scannmuster bestimmten x/y-Bearbeitungspunkt steuert der Schaltkreis 10 die Divergenzmodulation der Divergenzmodulatoren 121, 122, so dass mittels eines Laserpulses des Strahlteils L1 eine fokussierte Bearbeitung des Augengewebes 20 auf der unteren Aussenfläche 21u und mittels eines Laserpulses des Strahlteils L2 eine fokussierte Bearbeitung des Augengewebes 20 auf der oberen Aussenfläche 21o des Lentikels erfolgt, ohne dass dazu (ausser durch die Divergenzmodulatoren 121, 122) eine Änderung der Fokussierung respektive Fokussiervorrichtung 14 vorgenommen wird respektive werden muss.

In der Ausführungsvariante gemäss Figur 8, werden die Strahlteile L1, L2 ohne Divergenzmodulation durch die Divergenzmodulatoren 121, 122, also im Wesentlichen ohne unterschiedliche Divergenz erzeugt, und vertikal (in z-Richtung) mit derselben Tiefe auf dieselbe horizontale Bearbeitungsebene h fokussiert, jedoch aufgrund des Winkels zwischen den Strahlachsen der Strahlenbündel L1*, L2* respektive Strahlteile L1, L2 nicht auf dieselben Aussenflächen, sondern mit dem ersten Strahlenbündel L1* respektive Strahlteil L1 auf die untere Aussenfläche 21u und mit dem zweiten Strahlenbündel L2* respektive Strahlteil L2 auf die obere Aussenfläche 21o des zu schneidenden Lentikels 21 fokussiert. Durch Verstellen der Bearbeitungstiefe der Bearbeitungsebene h durch die Fokussieroptik 14 und des Winkels zwischen den Strahlachsen der Strahlenbündel L1*, L2* respektive Strahlteile L1, L2 durch den optischen Strahlkombinierer des Strahlvereinigers 125 gemäss der Figur 11 können die Foki der Strahlteile L1, L2 des Bearbeitungsstrahls dem Verlauf der unteren Aussenfläche 21u und der oberen Aussenfläche 21o nachgeführt werden und das Lentikel 21 durch Ablenken des Bearbeitungsstrahls mittels des Scannersystems 13 gemäss vorgegebenen Scannmustern geschnitten werden. Dabei wird das Strahlenbündel L1* respektive der Strahlteil L1 unterbunden, wenn die Bearbeitungstiefe der Bearbeitungsebene h über dem Apex A der unteren Aussenfläche 21u liegt, d.h. wenn das Strahlenbündel L1* respektive der Strahlteil L1 den Apex erreicht, und das Strahlenbündel L2* respektive der Strahlteil L2 wird alleine über den Bereich der oberen Aussenfläche 21o weitergeführt, der in z-Richtung über dem Apex A der unteren Aussenfläche 21u liegt.

Abschliessend soll festgehalten werden, dass die Ausführungsvariante gemäss der Figur 7 den Vorteil einer besonders einfachen Bauweise aufweist. Das Strahlteilsystem 12 umfasst einen Strahlteiler 124, der eingerichtet ist, den gepulsten Laserstrahl P in das erste Strahlenbündel L 1 * für den ersten Strahlteil L1 und in das zweite Strahlenbündel L2* für den zweiten Strahlteil L2 zu teilen; einen Divergenzmodulator 121 (oder 122), der eingerichtet ist, die Divergenz des ersten Strahlteils L1 (oder des zweiten Strahlteils L2) zu modulieren, so dass die beiden Strahlteile L1, L2 eine unterschiedliche Divergenz aufweisen, derart dass der erste Strahlteil L1 durch die Fokussieroptik 14 auf die erste Aussenfläche 21u und der zweite Strahlteil L2 auf die zweite Aussenfläche 21o des Lentikels 21 fokussiert wird; und einen Strahlvereiniger 125, der eingerichtet ist, das erste Strahlenbündel L1* und das zweite Strahlenbündel L2* mit einem Winkel zwischen den Strahlachsen der beiden Strahlenbündel L1*, L2* zum Bearbeitungslaserstrahl L zu vereinen, so dass die beiden Strahlteile L1, L2 mit unterschiedlichem Einstrahlwinkel auf die erste Aussenfläche 21u und die zweite Aussenfläche 21o des Lentikels 21 fokussiert werden. Durch den unterschiedlichen Einstrahlwinkel wird eine Abschattung und dadurch bewirkte Beeinflussung der beiden erzeugten Foki der Strahlteile L1, L2 vermieden.

## Patentansprüche

1. Ophthalmologische Vorrichtung (1) zum Bearbeiten von Augengewebe (20), umfassend eine Laserquelle (11), die eingerichtet ist, einen gepulsten Laserstrahl (P) zu erzeugen; eine Fokussieroptik (14), die eingerichtet ist, einen Bearbeitungslaserstrahl (L) ins Augengewebe (20) zu fokussieren, und ein Scannersystem (13), das eingerichtet ist, den Bearbeitungslaserstrahl (L) gemäss vorgegebenen Scannmustern ins Augengewebe (20) zu lenken; weiterhin umfassend ein Strahlteilsystem (12), das dem Scannersystem (13) vorgeschaltet ist und das eingerichtet ist, aus dem gepulsten Laserstrahl (P) den Bearbeitungslaserstrahl (L) derart zu erzeugen, dass der Bearbeitungslaserstrahl (L) einen ersten Strahlteil (L1) und einen zweiten Strahlteil (L2) umfasst, wobei der erste Strahlteil (L1) durch die Fokussieroptik (14) auf eine erste Aussenfläche (21u) eines im Augengewebe (20) zu schneidenden Lentikels (21) fokussiert wird und der zweite Strahlteil (L2) auf eine andere zweite Aussenfläche (21o) des im Augengewebe (20) zu schneidenden Lentikels (21) fokussiert wird, und dass bei einem Lenken des Bearbeitungslaserstrahl (L) ins Augengewebe (20) durch das Scannersystem (13) die erste Aussenfläche (21u) durch den ersten Strahlteil (L1) bearbeitet wird und die zweite Aussenfläche (21o) durch den zweiten Strahlteil (L2) bearbeitet wird.

2. Ophthalmologische Vorrichtung (1) nach Anspruch 1, wobei das Strahlteilsystem (12) einen dem Scannersystem (13) vorgeschalteten Divergenzmodulator (121) umfasst, der eingerichtet ist, eine Divergenz des ersten Strahlteils (L1) oder des zweiten Strahlteils (L2) zu modulieren, so dass der erste Strahlteil (L1) und der zweite Strahlteil (L2) eine unterschiedliche Divergenz aufweisen, derart dass der erste Strahlteil (L1) durch die Fokussieroptik (14) auf die erste Aussenfläche (21u) fokussiert wird und der zweite Strahlteil (L2) auf die zweite Aussenfläche (21o) fokussiert wird.

3. Ophthalmologische Vorrichtung (1) nach Anspruch 1, wobei das Strahlteilsystem (12) einen ersten Divergenzmodulator (121) umfasst, der eingerichtet ist, den ersten Strahlteil (L1) mit einer ersten Divergenz zu erzeugen; und das Strahlteilsystem (12) einen zweiten Divergenzmodulator (122) umfasst, der eingerichtet ist, den zweiten Strahlteil (L2) mit einer von der ersten Divergenz verschiedenen zweiten Divergenz zu erzeugen.

4. Ophthalmologische Vorrichtung (1) nach Anspruch 3, wobei die Fokussieroptik (14) eingerichtet ist, den Bearbeitungslaserstrahl (L) im Augengewebe (20) auf eine eingestellte Schnittposition zu fokussieren, der erste Divergenzmodulator (121) eingerichtet ist, den ersten Strahlteil (L1) derart mit der ersten Divergenz zu erzeugen, dass der erste Strahlteil (L1) durch die Fokussieroptik (14) im Augengewebe relativ zur eingestellten Schnittposition auf die erste Aussenfläche (21u) fokussiert wird, und der zweite Divergenzmodulator (122) eingerichtet ist, den zweiten Strahlteil (L2) derart mit der zweiten Divergenz zu erzeugen, dass der zweite Strahlteil (L2) durch die Fokussieroptik (14) im Augengewebe relativ zur eingestellten Schnittposition auf die zweite Aussenfläche (21o) fokussiert wird.

5. Ophthalmologische Vorrichtung (1) nach einem der Ansprüche 1 bis 4, wobei das Strahlteilsystem (12) eingerichtet ist, den gepulsten Laserstrahl (P) in ein erstes Strahlenbündel (L1*) und in ein zweites Strahlenbündel (L2^{∗}) zu teilen.

6. Ophthalmologische Vorrichtung (1) nach Anspruch 5, wobei das Strahlteilsystem (12) eingerichtet ist, einen ersten Puls des gepulsten Laserstrahls (P) dem ersten Strahlenbündel (L1*) zuzuführen, und einen dem ersten Puls unmittelbar folgenden zweiten Puls des gepulsten Laserstrahls (P) dem zweiten Strahlenbündel (L2*) zuzuführen.

7. Ophthalmologische Vorrichtung (1) nach Anspruch 6, wobei das Strahlteilsystem (12) einen optischen Modulator umfasst, der synchronisiert mit der Laserquelle (11) gekoppelt ist und eingerichtet ist, den ersten Puls des gepulsten Laserstrahls (P) dem ersten Strahlenbündel (L1^{∗}) zuzuführen, und den zweiten Puls des gepulsten Laserstrahls (P) dem zweiten Strahlenbündel (L2*) zuzuführen.

8. Ophthalmologische Vorrichtung (1) nach Anspruch 6, wobei das Strahlteilsystem (12) einen optischen Strahlteiler umfasst, der eingerichtet ist, den gepulsten Laserstrahl (P) in das erste Strahlenbündel (L1*) und in das zweite Strahlenbündel (L2*) aufzuteilen, das Strahlteilsystem (12) einen ersten optischen Modulator umfasst, der synchronisiert mit der Laserquelle (11) gekoppelt im ersten Strahlenbündel (L1*) angeordnet ist und eingerichtet ist, den zweiten Puls des gepulsten Laserstrahls (P) im ersten Strahlenbündel (L1*) auszublenden, und das Strahlteilsystem (12) einen zweiten optischen Modulator umfasst, der synchronisiert mit der Laserquelle (11) gekoppelt im zweiten Strahlenbündel (L2*) angeordnet ist und eingerichtet ist, den ersten Puls des gepulsten Laserstrahls (P) im zweiten Strahlenbündel (L2*) auszublenden.

9. Ophthalmologische Vorrichtung (1) nach einem der Ansprüche 5 bis 8, wobei das Strahlteilsystem (12) einen Strahlvereiniger (125) umfasst, der eingerichtet ist, das erste Strahlenbündel (L1*) und das zweite Strahlenbündel (L2*) zum Bearbeitungslaserstrahl (L) zu vereinen.

10. Ophthalmologische Vorrichtung (1) nach Anspruch 9, wobei der Strahlvereiniger (125) eingerichtet ist, das erste Strahlenbündel (L1*) und das zweite Strahlenbündel (L2*) mit einem Winkel zwischen einer Strahlachse des ersten Strahlenbündels (L1*) und einer Strahlachse des zweiten Strahlenbündels (L2*) zum Bearbeitungslaserstrahl (L) zu vereinen.

11. Ophthalmologische Vorrichtung (1) nach Anspruch 9, wobei der Strahlvereiniger (125) einen Strahlquerschnittvereiniger (123) umfasst, der eingerichtet ist, einen ersten Querschnittsbereich (W11) des ersten Strahlenbündels (L1*) einem ersten Querschnittsbereich (W10) des Bearbeitungslaserstrahls (L) zuzuführen, und einen zweiten Querschnittsbereich (W21) des zweiten Strahlenbündels (L2*) einem zweiten Querschnittsbereich (W20) des Bearbeitungslaserstrahls (L) zuzuführen, wobei sich der erste Querschnittsbereich (W10) des Bearbeitungslaserstrahls (L) und der zweite Querschnittsbereich (W20) des Bearbeitungslaserstrahls (L) nicht überschneiden.

12. Ophthalmologische Vorrichtung (1) nach einem der Ansprüche 1 bis 4, wobei das Strahlteilsystem (12) einen Strahlquerschnittteiler (120) umfasst, der eingerichtet ist, einen ersten Querschnittsbereich (W1) des gepulsten Laserstrahls (P) dem ersten Strahlteil (L1) zuzuführen, und einen zweiten Querschnittsbereich (W2) des gepulsten Laserstrahls (P) dem zweiten Strahlteil (L2) zuzuführen, wobei sich der erste Querschnittsbereich (W10) und der zweite Querschnittsbereich (W20) nicht überlappen.

13. Ophthalmologische Vorrichtung (1) nach Anspruch 12, wobei das Strahlteilsystem (12) einen Strahlquerschnittvereiniger (123) umfasst, der eingerichtet ist, den ersten Querschnittsbereich (W1) des gepulsten Laserstrahls (P) einem ersten Querschnittsbereich (W10) des Bearbeitungslaserstrahls (L) zuzuführen, und den zweiten Querschnittsbereich (W2) des gepulsten Laserstrahls (P) einem zweiten Querschnittsbereich (W20) des Bearbeitungslaserstrahls (L) zuzuführen, wobei sich der erste Querschnittsbereich (W10) des Bearbeitungslaserstrahls (L) und der zweite Querschnittsbereich (W20) des Bearbeitungslaserstrahls (L) nicht überschneiden.

14. Ophthalmologische Vorrichtung (1) nach einem der Ansprüche 1 bis 13, wobei das Scannersystem (13) eingerichtet ist, den Bearbeitungslaserstrahl (L) gemäss einem vorgegebenen Schnittlinien-Scannmuster ins Augengewebe (20) zu lenken, wobei der erste Strahlteil (L1) eine erste Schnittlinie auf der ersten Aussenfläche (21u) des Lentikels (21) erzeugt und der zweite Strahlteil (L2) eine zweite Schnittlinie auf der zweiten Aussenfläche (21o) des Lentikels (21) erzeugt.

15. Ophthalmologische Vorrichtung (1) nach Anspruch 14, wobei das Scannersystem (13) ein erstes Scannermodul umfasst, das eingerichtet ist, den Bearbeitungslaserstrahl (L) gemäss einer ersten Komponente des vorgegebenen Schnittlinien-Scannmusters in einer Vorschubrichtung entlang einer Bearbeitungslinie zu führen, und das Scannersystem (13) ein zweites Scannermodul umfasst, das eingerichtet ist, den Bearbeitungslaserstrahl (L) gemäss einer der ersten Komponente überlagerten zweiten Komponente des vorgegebenen Schnittlinien-Scannmusters entlang einer quer zur Bearbeitungslinie verlaufenden Scannlinie zu lenken, mit einer wesentlich höheren Scanngeschwindigkeit im Vergleich zur Scanngeschwindigkeit des ersten Scannermoduls in Vorschubrichtung, wobei der erste Strahlteil (L1) eine erste Schnittlinie mit der ersten Komponente und der überlagerten zweiten Komponente auf der ersten Aussenfläche (21u) des Lentikels (21) erzeugt und der zweite Strahlteil (L2) eine zweite Schnittlinie mit der ersten Komponente und der überlagerten zweiten Komponente auf der zweiten Aussenfläche (21o) des Lentikels (21) erzeugt.

16. Ophthalmologische Vorrichtung (1) nach einem der Ansprüche 1 bis 15, wobei das Strahlteilsystem (12) eingerichtet ist, den ersten Strahlteil (L1) und den zweiten Strahlteil (L2) derart zu erzeugen, dass der erste Strahlteil (L1) durch die Fokussieroptik (14) auf die erste Aussenfläche (21u) fokussiert wird, die weiter von der Fokussieroptik (14) entfernt ist als die zweite Aussenfläche (21o), auf die der zweite Strahlteil (L1) durch die Fokussieroptik (14) fokussiert wird.

## Claims

1. Ophthalmological device (1) for processing eye tissue (20), comprising a laser source (11) configured to generate a pulsed laser beam (P); a focusing optical unit (14) configured to focus a processing laser beam (L) into the eye tissue (20), and a scanner system (13) configured to direct the processing laser beam (L) into the eye tissue (20) in accordance with predefined scanning patterns; furthermore comprising a beam splitting system (12) disposed upstream of the scanner system (13) and configured to generate the processing laser beam (L) from the pulsed laser beam (P) in such a way that the processing laser beam (L) comprises a first beam part (L1) and a second beam part (L2), wherein the first beam part (L1) is focused by the focusing optical unit (14) onto a first outer surface (21u) of a lenticule (21) to be cut in the eye tissue (20), and the second beam part (L2) is focused onto a different, second outer surface (21o) of the lenticule (21) to be cut in the eye tissue (20), and that, in the course of directing the processing laser beam (L) into the eye tissue (20) by means of the scanner system (13), the first outer surface (21u) is processed by the first beam part (L1) and the second outer surface (21o) is processed by the second beam part (L2).

2. Ophthalmological device (1) according to Claim 1, wherein the beam splitting system (12) comprises a divergence modulator (121) disposed upstream of the scanner system (13) and configured to modulate a divergence of the first beam part (L1) or of the second beam part (L2), such that the first beam part (L1) and the second beam part (L2) have a different divergence, in such a way that the first beam part (L1) is focused onto the first outer surface (21u) by the focusing optical unit (14) and the second beam part (L2) is focused onto the second outer surface (21o).

3. Ophthalmological device (1) according to Claim 1, wherein the beam splitting system (12) comprises a first divergence modulator (121) configured to generate the first beam part (L1) with a first divergence; and the beam splitting system (12) comprises a second divergence modulator (122) configured to generate the second beam part (L2) with a second divergence, which is different from the first divergence.

4. Ophthalmological device (1) according to Claim 3, wherein the focusing optical unit (14) is configured to focus the processing laser beam (L) in the eye tissue (20) onto a set cutting position, the first divergence modulator (121) is configured to generate the first beam part (L1) with the first divergence in such a way that the first beam part (L1) is focused in the eye tissue with respect to the set cutting position onto the first outer surface (21u) by the focusing optical unit (14), and the second divergence modulator (122) is configured to generate the second beam part (L2) with the second divergence in such a way that the second beam part (L2) is focused in the eye tissue with respect to the set cutting position onto the second outer surface (21o) by the focusing optical unit (14).

5. Ophthalmological device (1) according to any of Claims 1 to 4, wherein the beam splitting system (12) is configured to split the pulsed laser beam (P) into a first beam bundle (L1*) and into a second beam bundle (L2*) .

6. Ophthalmological device (1) according to Claim 5, wherein the beam splitting system (12) is configured to feed a first pulse of the pulsed laser beam (P) to the first beam bundle (L1*), and to feed a second pulse of the pulsed laser beam (P), said second pulse directly succeeding the first pulse, to the second beam bundle (L2*) .

7. Ophthalmological device (1) according to Claim 6, wherein the beam splitting system (12) comprises an optical modulator, which is coupled to the laser source (11) in a synchronized manner and is configured to feed the first pulse of the pulsed laser beam (P) to the first beam bundle (L1*), and to feed the second pulse of the pulsed laser beam (P) to the second beam bundle (L2*).

8. Ophthalmological device (1) according to Claim 6, wherein the beam splitting system (12) comprises an optical beam splitter configured to split the pulsed laser beam (P) into the first beam bundle (L1*) and into the second beam bundle (L2*), the beam splitting system (12) comprises a first optical modulator, which is arranged in the first beam bundle (L1*) in a manner coupled to the laser source (11) in a synchronized manner and is configured to mask out the second pulse of the pulsed laser beam (P) in the first beam bundle (L1*), and the beam splitting system (12) comprises a second optical modulator, which is arranged in the second beam bundle (L2*) in a manner coupled to the laser source (11) in a synchronized manner and is configured to mask out the first pulse of the pulsed laser beam (P) in the second beam bundle (L2*).

9. Ophthalmological device (1) according to any of Claims 5 to 8, wherein the beam splitting system (12) comprises a beam combiner (125) configured to combine the first beam bundle (L1*) and the second beam bundle (L2*) to form the processing laser beam (L).

10. Ophthalmological device (1) according to Claim 9, wherein the beam combiner (125) is configured to combine the first beam bundle (L1*) and the second beam bundle (L2*) to form the processing laser beam (L) with an angle between a beam axis of the first beam bundle (L1*) and a beam axis of the second beam bundle (L2*).

11. Ophthalmological device (1) according to Claim 9, wherein the beam combiner (125) comprises a beam cross section combiner (123) configured to feed a first cross-sectional region (W11) of the first beam bundle (L1*) to a first cross-sectional region (W10) of the processing laser beam (L), and to feed a second cross-sectional region (W21) of the second beam bundle (L2*) to a second cross-sectional region (W20) of the processing laser beam (L), wherein the first cross-sectional region (W10) of the processing laser beam (L) and the second cross-sectional region (W20) of the processing laser beam (L) do not overlap.

12. Ophthalmological device (1) according to any of Claims 1 to 4, wherein the beam splitting system (12) comprises a beam cross section splitter (120) configured to feed a first cross-sectional region (W1) of the pulsed laser beam (P) to the first beam part (L1), and to feed a second cross-sectional region (W2) of the pulsed laser beam (P) to the second beam part (L2), wherein the first cross section region (W10) and the second cross-sectional region (W20) do not overlap.

13. Ophthalmological device (1) according to Claim 12, wherein the beam splitting system (12) comprises a beam cross section combiner (123) configured to feed the first cross-sectional region (W1) of the pulsed laser beam (P) to a first cross-sectional region (W10) of the processing laser beam (L), and to feed the second cross-sectional region (W2) of the pulsed laser beam (P) to a second cross-sectional region (W20) of the processing laser beam (L), wherein the first cross-sectional region (W10) of the processing laser beam (L) and the second cross-sectional region (W20) of the processing laser beam (L) do not overlap.

14. Ophthalmological device (1) according to any of Claims 1 to 13, wherein the scanner system (13) is configured to direct the processing laser beam (L) into the eye tissue (20) in accordance with a predefined cutting line scanning pattern, wherein the first beam part (L1) generates a first cutting line on the first outer surface (21u) of the lenticule (21) and the second beam part (L2) generates a second cutting line on the second outer surface (21o) of the lenticule (21).

15. Ophthalmological device (1) according to Claim 14, wherein the scanner system (13) comprises a first scanner module configured to guide the processing laser beam (L) in accordance with a first component of the predefined cutting line scanning pattern in a feed direction along a processing line, and the scanner system (13) comprises a second scanner module configured to direct the processing laser beam (L) in accordance with a second component of the predefined cutting line scanning pattern, said second component being superimposed on the first component, along a scanning line running transversely with respect to the processing line, at a significantly higher scanning speed in comparison with the scanning speed of the first scanner module in the feed direction, wherein the first beam part (L1) generates a first cutting line having the first component and the superimposed second component on the first outer surface (21u) of the lenticule (21) and the second beam part (L2) generates a second cutting line having the first component and the superimposed second component on the second outer surface (21o) of the lenticule (21).

16. Ophthalmological device (1) according to any of Claims 1 to 15, wherein the beam splitting system (12) is configured to generate the first beam part (L1) and the second beam part (L2) in such a way that the first beam part (L1) is focused by the focusing optical unit (14) onto the first outer surface (21u), which is further away from the focusing optical unit (14) than the second outer surface (21o), onto which the second beam part (L1) is focused by the focusing optical unit (14).

## Revendications

1. Dispositif ophtalmologique (1) pour traiter du tissu oculaire (20), ledit dispositif comprenant une source laser (11) qui est conçue pour générer un rayon laser pulsé (P); une optique de focalisation (14) qui est conçue pour focaliser un rayon laser de traitement (L) dans le tissu oculaire (20), et un système de balayage (13) qui est conçu pour diriger le rayon laser de traitement (L) dans le tissu oculaire (20) selon des motifs de balayage spécifiés; ledit dispositif comprenant en outre un système de séparation de rayon (12) qui est placé en amont du système de balayage (13) et qui est conçu pour générer le rayon laser de traitement (L) à partir du rayon laser pulsé (P) de manière que le rayon laser de traitement (L) comprenne une première partie de rayon (L1) et une deuxième partie de rayon (L2), la première partie de rayon (L1) étant focalisée par l'optique de focalisation (14) sur une première surface extérieure (21u) d'une lenticule (21) à découper dans le tissu oculaire (20) et la deuxième partie de rayon (L2) étant focalisée sur une autre deuxième surface extérieure (21o) de la lenticule (21) à découper dans le tissu oculaire (20) et, lorsque le rayon laser de traitement (L) est dirigé dans le tissu oculaire (20) par le système de balayage (13), la première surface extérieure (21u) étant traitée par la première partie de rayon (L1) et la deuxième surface extérieure (21o) étant traitée par la deuxième partie de rayon (L2).

2. Dispositif ophtalmologique (1) selon la revendication 1, le système de séparation de rayon (12) comprenant un modulateur de divergence (121) qui est placé en amont du système de balayage (13) et qui est conçu pour moduler une divergence de la première partie de rayon (L1) ou de la deuxième partie de rayon (L2) de sorte que la première partie de rayon (L1) et la deuxième partie de rayon (L2) aient une divergence différente de manière que la première partie de rayon (L1) soit focalisée par l'optique de focalisation (14) sur la première surface extérieure (21u) et la deuxième partie de rayon (L2) soit focalisée sur la deuxième surface extérieure (21o).

3. Dispositif ophtalmologique (1) selon la revendication 1, le système de séparation de rayon (12) comprenant un premier modulateur de divergence (121) qui est conçu pour générer la première partie de rayon (L1) avec une première divergence; et le système de séparation de rayon (12) comprenant un deuxième modulateur de divergence (122) conçu pour générer la deuxième partie de rayon (L2) avec une deuxième divergence différente de la première divergence.

4. Dispositif ophtalmologique (1) selon la revendication 3, l'optique de focalisation (14) étant conçue pour focaliser le rayon laser de traitement (L) dans le tissu oculaire (20) sur une position d'incision réglée, le premier modulateur de divergence (121) étant conçu pour générer la première partie de rayon (L1) avec la première divergence de manière que la première partie de rayon (L1) soit focalisée par l'optique de focalisation (14) dans le tissu oculaire par rapport à la position d'incision réglée sur la première surface extérieure (21u), et le deuxième modulateur de divergence (122) étant conçu pour générer la deuxième partie de rayon (L2) avec la deuxième divergence de manière que la deuxième partie de rayon (L2) soit focalisée par l'optique de focalisation (14) dans le tissu oculaire par rapport à la position d'incision réglée sur la deuxième surface extérieure (21o).

5. Dispositif ophtalmologique (1) selon l'une des revendications 1 à 4, le système de séparation de rayon (12) étant conçu pour séparer le rayon laser pulsé (P) en un premier faisceau de rayons (L1*) et en un deuxième faisceau de rayons (L2*).

6. Dispositif ophtalmologique (1) selon la revendication 5, le système de séparation de rayon (12) étant conçu pour amener une première impulsion du rayon laser pulsé (P) au premier faisceau de rayons (L1*), et pour amener une deuxième impulsion du rayon laser pulsé (P), suivant immédiatement la première impulsion, au deuxième faisceau de rayons (L2*).

7. Dispositif ophtalmologique (1) selon la revendication 6, le système de séparation de rayon (12) comprenant un modulateur optique qui est couplé de manière synchronisée à la source laser (11) et qui est conçu pour amener la première impulsion du rayon laser pulsé (P) au premier faisceau de rayons (L1*) et la deuxième impulsion du rayon laser pulsé (P) au deuxième faisceau de rayons (L2*).

8. Dispositif ophtalmologique (1) selon la revendication 6, le système de séparation de rayon (12) comprenant un séparateur de rayon optique qui est conçu pour séparer le rayon laser pulsé (P) en le premier faisceau de rayons (L1*) et en le deuxième faisceau de rayons (L2*), le système de séparation de rayon (12) comprenant un premier modulateur optique qui est disposé dans le premier faisceau de rayons (L1*) et couplé de manière synchronisée à la source laser (11) et étant conçu pour masquer la deuxième impulsion du rayon laser pulsé (P) dans le premier faisceau de rayons (L1*), et le système de séparation de rayon (12) comprenant un deuxième modulateur optique qui est disposé dans le deuxième faisceau de rayons (L2*) et couplé de manière synchronisée à la source laser (11) et qui est conçu pour masquer la première impulsion du rayon laser pulsé (P) dans le deuxième faisceau de rayons (L2*).

9. Dispositif ophtalmologique (1) selon l'une des revendications 5 à 8, le système de séparation de rayon (12) comprenant un combinateur de rayons (125) qui est conçu pour combiner le premier faisceau de rayons (L1*) et le deuxième faisceau de rayons (L2*) afin de former le rayon laser de traitement (L).

10. Dispositif ophtalmologique (1) selon la revendication 9, le combinateur de rayons (125) étant conçu pour combiner le premier faisceau de rayons (L1*) et le deuxième faisceau de rayons (L2*) en formant un angle entre un axe de rayon du premier faisceau de rayons (L1*) et un axe de rayon du deuxième faisceau de rayons (L2*) afin de former le rayon laser de traitement (L).

11. Dispositif ophtalmologique (1) selon la revendication 9, le combinateur de rayons (125) comprenant un combinateur de section transversale de rayon (123) qui est conçu pour amener une première région de section transversale (W11) du premier faisceau de rayons (L1*) à une première région de section transversale (W10) du rayon laser de traitement (L), et pour amener une deuxième région de section transversale (W21) du deuxième faisceau de rayons (L2*) à une deuxième région de section transversale (W20) du rayon laser de traitement (L), la première région de section transversale (W10) du rayon laser de traitement (L) et la deuxième région de section transversale (W20) du rayon laser de traitement (L) ne se chevauchant pas.

12. Dispositif ophtalmologique (1) selon l'une des revendications 1 à 4, le système de séparation de rayon (12) comprenant un séparateur en section transversale de rayon (120) qui est conçu pour amener une première région de section transversale (W1) du rayon laser pulsé (P) à la première partie de rayon (L1), et une deuxième région de section transversale (W2) du rayon laser pulsé (P) à la deuxième partie de rayon (L2), la première région de section transversale (W10) et la deuxième région de section transversale (W20) ne se chevauchant pas.

13. Dispositif ophtalmologique (1) selon la revendication 12, le système de séparation de rayon (12) comprenant un combinateur de section transversale de rayon (123) qui est conçu pour amener la première région de section transversale (W1) du rayon laser pulsé (P) à une première région de section transversale (W10) du rayon laser de traitement (L) et pour amener la deuxième région de section transversale (W2) d'un rayon laser pulsé (P) à une deuxième région de section transversale (W20) du rayon laser de traitement (L), la première région de section transversale (W10) du rayon laser de traitement (L) et la deuxième région de section transversale (W20) du rayon laser de traitement (L) ne se chevauchant pas.

14. Dispositif ophtalmologique (1) selon l'une des revendications 1 à 13, le système de balayage (13) étant conçu pour diriger le rayon laser de traitement (L) dans le tissu oculaire (20) selon un motif de balayage de ligne de coupe spécifié, la première partie de rayon (L1) générant une première ligne de coupe sur la première surface extérieure (21u) de la lenticule (21) et la deuxième partie de rayon (L2) générant une deuxième ligne de coupe sur la deuxième surface extérieure (21o) de la lenticule (21).

15. Dispositif ophtalmologique (1) selon la revendication 14, le système de balayage (13) comprenant un premier module de balayage qui est conçu pour guider le rayon laser de traitement (L) dans une direction de l'avance le long d'une ligne de traitement selon une première composante du motif de balayage de ligne de coupe spécifiée, et le système de balayage (13) comprenant un deuxième module de balayage qui est conçu pour diriger le rayon laser de traitement (L) selon une deuxième composante, superposée à la première composante, du motif de balayage de ligne de coupe spécifié le long d'une ligne de balayage qui s'étend transversalement à la ligne de traitement, avec une vitesse de balayage nettement plus élevée par rapport à la vitesse de balayage du premier module de balayage dans la direction de l'avance, la première partie de rayon (L1) générant une première ligne d'intersection sur la première surface extérieure (21u) de la lenticule (21) avec la première composante et la deuxième composante superposée et la deuxième partie de rayon (L2) générant une deuxième ligne d'intersection sur la deuxième surface extérieure (21o) de la lenticule (21) avec la première composante et la deuxième composante superposée.

16. Dispositif ophtalmologique (1) selon l'une des revendications 1 à 15, le système de séparation de rayon (12) étant conçu pour générer la première partie de rayon (L1) et la deuxième partie de rayon (L2) de manière que la première partie de rayon (L1) soit focalisée par l'optique de focalisation (14) sur la première surface extérieure (21u) qui est plus éloignée de l'optique de focalisation (14) que la deuxième surface extérieure (21o) sur laquelle la deuxième partie de rayon (L1) est focalisée par l'optique de focalisation (14).
